(19) Europäisches Patentamt
European Patent Office
Office européen des brevets

(11) Publication number: **0 434 038 A1**

# (12) EUROPEAN PATENT APPLICATION

(21) Application number: **90124780.9**

(22) Date of filing: **19.12.90**

(51) Int. Cl.5: **C07D 471/04**, C07D 487/04, C07D 473/00, A61K 31/435, A61K 31/495

(30) Priority: **22.12.89 JP 333722/89**

(43) Date of publication of application: **26.06.91 Bulletin 91/26**

(84) Designated Contracting States: **AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Takehiko, Naka**
**15-711, 4 Kamokogahara 1-chome, Higashinada-ku**
**Kobe, Hyogo 658(JP)**
Inventor: **Kohei, Nishikawa**
**5-19, Oharano-kamisatotorimicho**
**Nishikyo-ku, Kyoto 610-11(JP)**

(74) Representative: **Lederer, Franz, Dr. et al**
**Lederer, Keller & Riederer, Patentanwälte,**
**Lucile-Grahn-Strasse 22**
**W-8000 München 80(DE)**

## (54) Fused imidazole derivatives, their production and use.

(57) Novel fused imidazole derivatives of the formula (I):

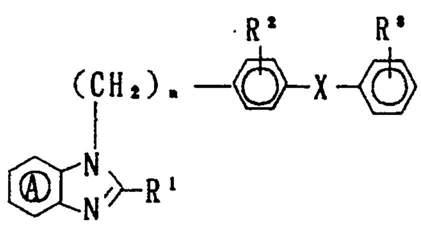

wherein the ring A, which may be optionally substituted, is a six-membered heteroaromatic group having one or two hetero nitrogen atoms; $R^1$ is hydrogen or an alkyl, aralkyl or aryl group which may be optionally substituted; $R^2$ is hydrogen, halogen or nitro; $R^3$ is a residue capable of forming an anion or a residue convertible into an anion; X is a direct bond or a spacer-having atomic length of two or less; and n is an integer of 1 or 2; and the pharmaceutically acceptable salts thereof have potent angiotensin II antagonist activity and hypotensive activity, thus being useful as therapeutic agents for treating circulatory system diseases such as hypertensive diseases, heart diseases, strokes, etc.

EP 0 434 038 A1

# FUSED IMIDAZOLE DERIVATIVES, THEIR PRODUCTION AND USE

## FIELD OF THE INVENTION

The present invention relates to novel fused imidazole derivatives having potent pharmacological activity and intermediates for the preparation thereof. More particularly, the present invention relates to compounds having potent angiotensin II antagonist activity
and hypotensive activity, which are useful as therapeutic agents for treating circulatory system diseases such as hypertensive diseases, heart diseases, strokes and the like.

## BACKGROUND OF THE INVENTION

The renin-angiotensin system is involved in the homeostatic function to control systemic blood pressure, the volume of body fluid, balance among the electrolytes, etc., associated with the aldosterone system. Development of angiotensin II converting enzyme inhibitors (ACE inhibitor) (this converting enzyme produces angiotensin II which possesses potent vasoconstrictive activity) has clarified the relation between the renin-angiotensin system and hypertension. Since angiotensin II elevates blood pressure via the angiotensin II receptors on cell membranes, angiotensin II antagonists as well as the ACE inhibitor would be useful in treating hypertension.

It has been reported that various angiotensin II analogues such as saralasin, [$Sar^1$,$Ile^8$]A II and the like possess potent angiotensin II antagonist activity.

It has, however, been reported that, when peptide antagonists are administered parenterally, their actions are not prolonged and, when administered orally, they are ineffective (M. A. Ondetti and D. W. Cushman, Annual Reports in Medicinal Chemistry, 13, 82-91 (1978)).

Non-peptide angiotensin II antagonists are disclosed in Japanese Patent Laid Open No. 71073/1981; No. 71074/1981; No. 92270/1982; No. 157768/1983; No. 23868/1988; No. 117876/1989, etc.

Imidazole derivatives having angiotensin II antagonist activity are disclosed in A. T. Chiu et al., Eur. J. Pharm., 157, 13 (1981), P. C. Wong et al., J. Pharmcol. Exp. Ther., 247, 1 (1988), P. C. Wong et al. , Hypertension, 13, 489 (1989), etc.

It has not yet been known that fused imidazole derivatives possess potent angiotensin II antagonist activity.

## SUMMARY OF THE INVENTION

The present inventors made extensive investigations to prepare useful compounds which have angiotensin II antagonist activity. As a result of these researches, the present inventors have succeeded in synthesizing fused imidazole derivatives possessing excellently potent angiotensin II antagonist activity and developed their work to accomplish the present invention.

The present invention provides fused imidazole derivatives having the formula I:

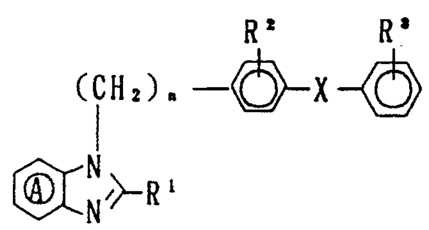

wherein the ring A, which may be optionally substituted, is a six-membered heteroaromatic group having one or two hetero nitrogen atoms; $R^1$ is hydrogen or an alkyl, aralkyl or aryl group which may be optionally substituted;

$R^2$ is hydrogen, halogen or nitro;

$R^3$ is a residue capable of forming an anion or a residue convertible into an anion;

X is a direct bond or a spacer having atomic length of two or less; and

n is an integer of 1 or 2;

and the pharmaceutically acceptable salts thereof.

With regard to the foregoing formula (I), alkyl groups for $R^1$ include lower alkyl of 1 to about 8 carbon atoms, which may be straight or branched, (e.g. methyl, ethyl, propyl, isopropyl, butyl, isobutyl, sec-butyl, t-butyl, pentyl, i-pentyl, hexyl, heptyl, octyl, and the like), and may be optionally substituted with hydroxyl, amino which may be optionally substituted, halogen, lower ($C_{1-4}$) alkoxy, and the like.

Aralkyl groups for $R^1$ include phenyl-lower ($C_{1-4}$) alkyl such as benzyl, phenethyl, phenylpropyl, and the like and may be optionally substituted with halogen (e.g. F, Cl, Br and the like), nitro, lower ($C_{1-4}$) alkoxy (e.g. methoxy, ethoxy, and the like), lower ($C_{1-4}$) alkyl (e.g. methyl, ethyl, and the like).

Aryl groups for $R^1$ include phenyl, naphtyl, and the like and may be optionally substituted with halogen (e.g. F, Cl, Br and the like), nitro, lower ($C_{1-4}$) alkoxy (e.g. methoxy, ethoxy, and the like), lower ($C_{1-4}$) alkyl (e.g. methyl, ethyl, and the like), and the like.

Six-membered heteroaromatic groups having one or two hetero nitrogen atoms for the ring A include a six-membered heteroaromatic group having one hetero nitrogen atom such as, for example, pyridine, a six-membered heteroaromatic group having two hetero nitrogen atoms such as, for example, pyrazine, pyrimidine, pyridazine, and the like and are preferably pyridine and pyrazine.

The ring A may be optionally substituted with, for example, halogen (e.g. F, Cl, Br and the like); nitro; cyano; optionally substituted amino (e.g. amino, N-lower ($C_{1-4}$) alkyl amino (e.g. methylamino, and the like), N,N-dilower ($C_{1-4}$) alkyl amino (e.g. dimethylamino, and the like), N-arylamino (e.g. phenylamino, and the like), alicyclic amino (e.g. morpholino, piperidino, piperazino, N-phenylpiperazino, and the like), and the like); -Y-R wherein Y is a bond, -O-, -S- or -(C=O)-, and R is lower alkyl which may be optionally substituted (e.g. lower ($C_{1-4}$) alkyl which may be optionally substituted with hydroxyl, amino which may be optionally substituted, halogen and lower ($C_{1-4}$) alkoxy; or -COD wherein D is lower ($C_{1-4}$) alkoxy which may be optionally substituted (e.g. a lower ($C_{1-4}$) alkoxy group which may be optionally substituted with hydroxyl, optionally substituted amino, halogen and lower ($C_{1-4}$) alkoxy on the alkyl portion), optionally substituted amino [e.g. amino, N-lower ($C_{1-4}$) alkyl amino (e.g. methylamino, and the like), N,N-dilower ($C_{1-4}$) alkyl amino (e.g. dimethylamino, and the like), N-arylamino (e.g. phenylamino, and the like), alicyclic amino (e.g. morpholino, piperidino, piperazino, N-phenylpiperazino, and the like), and the like], halogen (e.g. Cl and the like) or OH, and the like.

The nitrogen atoms in the ring A may be optionally oxidized to form an N-oxide.

The compounds wherein the ring A is substituted with -COD wherein D is halogen are useful as synthetic intermediates for the preparation of the compounds wherein the ring A is substituted with -COD wherein D is lower ($C_{1-4}$) alkoxy which may be optionally substituted or amino which may be optionally substituted.

$R^2$ represents hydrogen, halogen (e.g. Cl, Br and the like), or nitro and may be in the ortho or meta position.

Examples of residues capable of forming an anion and residues convertible into the anion for $R^3$ include carboxyl, lower ($C_{1-4}$) alkoxycarbonyl, cyano, tetrazolyl, trifluoromethanesulfonic amide ($-NHSO_2CF_3$), phosphoric acid, sulfonic acid, and the like.

Such residues may include those which are capable of forming anions either chemically or under biological and/or physiological conditions.

$R^3$ may be in the ortho, meta or para position, and preferably is in the ortho position.

The compounds wherein $R^3$ is a residue capable of forming an anion or convertible thereinto chemically (e.g. by oxidation, reduction or hydrolysis), (e.g. cyano and the like) are useful as synthetic intermediates.

The group X represents a direct bond or a spacer having atomic length of two or less between a phenylene group and a phenyl group. Examples of spacers having atomic length of two or less include divalent chains wherein the constituent number of atoms in a straight chain is 1 or 2 and which may be branched. Examples of such spacers include lower ($C_{1-4}$) alkylene, -C(=O)-, -O-, -S-, -NH-, -C(=O)-NH-, $-O-CH_2-$, $-S-CH_2-$, -CH=CH-, etc.

A preferred embodiment of the invention is a compound of the formula (I')

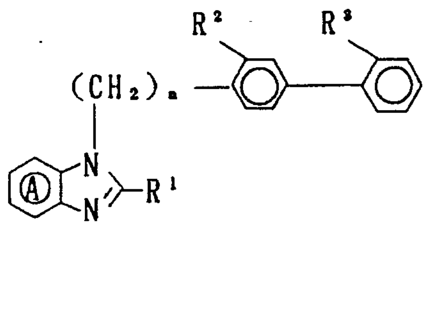

(I')

wherein the ring A is pyridine or pyrazine, which may be optionally substituted with one to three halogens (preferably one halogen, more preferably Cl); lower ($C_{1-4}$) alkoxy; nitro; amino, N-lower ($C_{1-4}$) alkyl amino, N,N-dilower ($C_{1-4}$) alkyl amino or -COD wherein D is hydroxyl or lower ($C_{1-4}$) alkoxy; $R^1$ is $C_{2-6}$alkyl; $R^2$ is hydrogen; and $R^3$ is carboxyl or tetrazolyl; and the pharmaceutically acceptable salts thereof.

The compounds (I) of the present invention may be prepared by several reaction schemes, as illustrated below for a preferred compound.

Scheme A

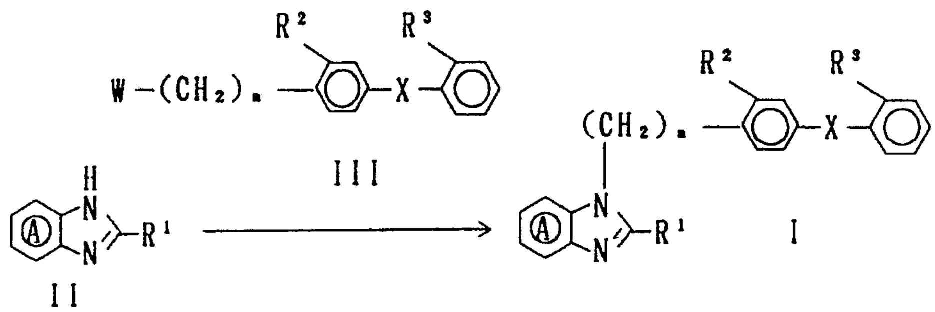

wherein A, $R^1$, $R^2$, $R^3$, X and n have the above-defined meanings and W is halogen.

Scheme B

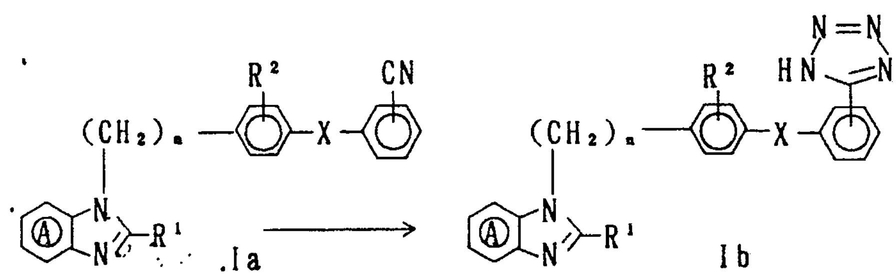

wherein each group has the above-defined meaning.

Scheme C

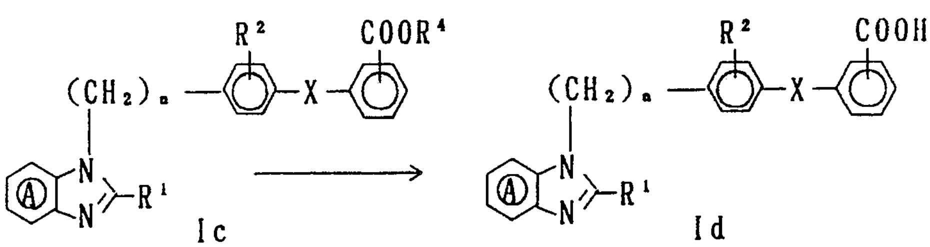

wherein A, $R^1$, $R^2$, X and n have the above-defined meanings and $R^4$ is lower ($C_{1-4}$) alkyl which may be optionally substituted.

Scheme D

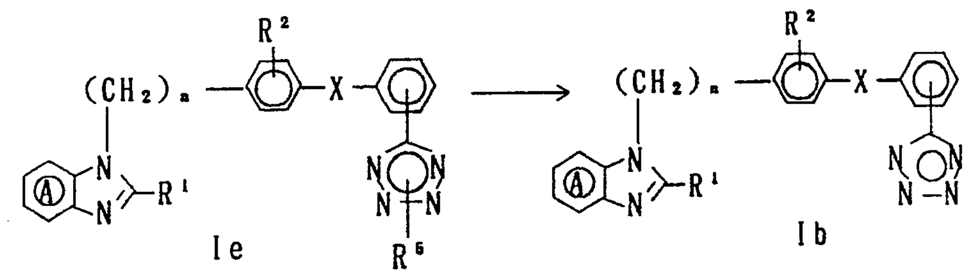

wherein A, $R^1$, $R^2$, X and n have the above-defined meanings and $R^5$ is an acid-labile protective group (e.g. triphenylmethyl, methoxymethyl).

The reaction as illustrated in Scheme A is an alkylation using an alkylating agent in the presence of a base. One molar portion of the compound (II) is employed with 1 to 3 moles of the base and about 1 to about 3 moles of the alkylating agent (III). The reaction is conventionally conducted in solvents such as dimethylformamide, dimethylacetamide, dimethylsulfoxide, acetonitrile, acetone, ethylmethylketone, and the like. Examples of such bases include sodium hydride, potassium t-butoxide, potassium carbonate, sodium carbonate, and the like. Examples of such alkylating agents (III) include substituted halides (e.g. chlorides, bromides, iodides, and the like), substituted sulfonate esters (e.g. methyl p-toluene-sulfonate esters, and the like), etc.

The reaction conditions may vary depending on the combination of the base and the alkylating agent (III). A temperature in the range of from ice-cooling to room temperature is preferred and a reaction period of from about 1 to about 10 hours is preferably employed.

The cyano substituent on the benzene of the componds (Ia) is reacted with various azides to form the tetrazole componds (Ib) as illustrated in Scheme B. One molar portion of the compound (Ia) is employed with about 1 to about 3 moles of the azide. The reaction is conventionally conducted in solvents such as dimethylformamide, dimethylacetamide, toluene, benzene, and the like.

Examples of such azides include trialkyl-tin azide, triphenyl-tin azide, hydrogen azide, and the like. In the case wherein the organo-tin azide compound is employed, the reaction is carried out in toluene or benzene by heating under a reflux for a period of from about 10 to about 30 hours. When the hydrogen azide is used, 2 moles of sodium azide and ammonium chloride per compound (Ib) are employed and the reaction is conducted in dimethylformamide at a temperature of from about 100°C to about 130°C for 1 to 3 days. During this reaction, it is preferable to facilitate working by adding an appropriate amount of sodium azide and ammonium chloride.

The reaction as illustrated in Scheme C is hydrolysis of the ester (Ic) into the carboxylic acid (Id) in the presence of an alkali. One molar portion of the compound (Ic) is employed with 1 to 3 moles of the alkali. The reaction is conventionally conducted in solvents such as alcohols containing water (e.g. methanol, ethanol, methylcellosolve, and the like). Examples of such alkalis include sodium hydroxide, potassium hydroxide, and the like. The reaction is preferably conducted at a temperature in the range from room temperature to 100°C for a period from about 1 to about 10 hours.

The reaction as illustrated in Scheme D is hydrolysis of the protected tetrazole derivative into the deprotected compound (Ib) in the presence of an acid. One molar portion of the compound (Ie) is employed with 1 to excess moles of the acid. The reaction is conventionally conducted in solvents such as alcohols containing water (e.g. methanol, ethanol, methylcellosolve, and the like). Examples of such acids include acetic acid, hydrochloric acid, and the like. The reaction is preferably conducted at a temperature in the range from 0°C to 50°C for a period from about 1 to about 10 hours.

The compounds (I) thus produced via the reaction processes as depicted in Schemes A, B, C and D can be isolated and purified from the reaction mixture according to conventional methods such as, for example, evaporation of solvents, extraction by water or organic solvents, concentration, neutralization, recrystallization, distillation, column chromatography and the like, to obtain a crystalline or oily product.

The compounds (I) of the present invention can be used in the form of salts derived from pharmaceutically or physiologically acceptable acids or bases. These salts include but are not limited to the following: salts with inorganic acids such as hydrochloric acid, sulphuric acid, nitric acid, phosphoric acid and, as the case may be, such organic acids as acetic acid, oxalic acid, succinic acid, maleic acid. Other salts include salts with alkali metals or alkaline earth metals, such as sodium, potassium, calcium or magnesium or with organic bases.

The starting materials (II) may be prepared by or according to methods described in, for example,

(1) G. W. H. Cheeseman and R. F. Cookson, The Chemistry of Heterocyclic Compounds, Condensed Pyrazines, Vol. 35, Ed. by A. Weissberger and E. C. Taylor, John Wiley & Sons, New York (1979), pp. 375-379,

(2) R. A. Abramovitch and J. G. Saha, Advance in Heterocyclic Chemistry, Pyrazine, Vol. 14, Ed. by A. R. Katritzky and A. J. Boulton, Acad. Press. Inc., New York and London (1966), pp. 99,

(3) The Chemistry of Heterocyclic Compounds, Pyrazine and Its Derivatives, Part 1-4, Ed. by R. A. Abramovitch et al., Interscience Publishers Inc., New York (1962),

(4) Y. C. Tong, J. Heterocycl. Chem., 12, 1127 (1975),

(5) M. Israel and A. R. Dar, J. Org. Chem., 24, 1455 (1959),

(6) H. Vorbruggen and K. Krolikiewicz, Synthesis, 1983, 316,

(7) P. Kumar and C. V. Ratnam, Indian J. Chem., 160, 531 (1978), and

(8) W. V. Bedenburg, G.Steinmetz and K. Thiele, Chem. Ztg. 103, 387 (1979).

The compounds (IIIa) are commercially available or easily prepared by halogenomethylation according to methods described in known literatures such as, for example,

(1) J. R. E. Hoover, A. W. Chow, R. J. Stedman, N. M. Hall, H. S. Greenberg, M. M. Dolan and R. J. Feriauto, J. Med. Chem., 7, 245 (1964),

(2) R. J. Stedman, J. R. E. Hoover, A. W. Chow, M. M. Dolan, N. M. Hall and R. J. Feriauto, J. Med. Chem., 7, 251 (1964),

(3) H. Gilman and R. D. Gorsich, J. Am. Chem. Soc., 78, 2217 (1956), and

(4) M. Orchin and E. Oscar Woolfolk, J. Am. Chem. Soc., 67, 122 (1945).

Scheme E

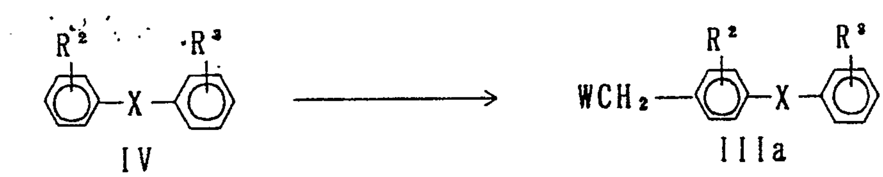

IV IIIa

wherein each group has the above-defined meaning.

The compounds (III) wherein n is 2 (the compounds (IIIb)) can also be easily prepared from the compounds (IIIa) as illustrated in Scheme F.

Scheme F

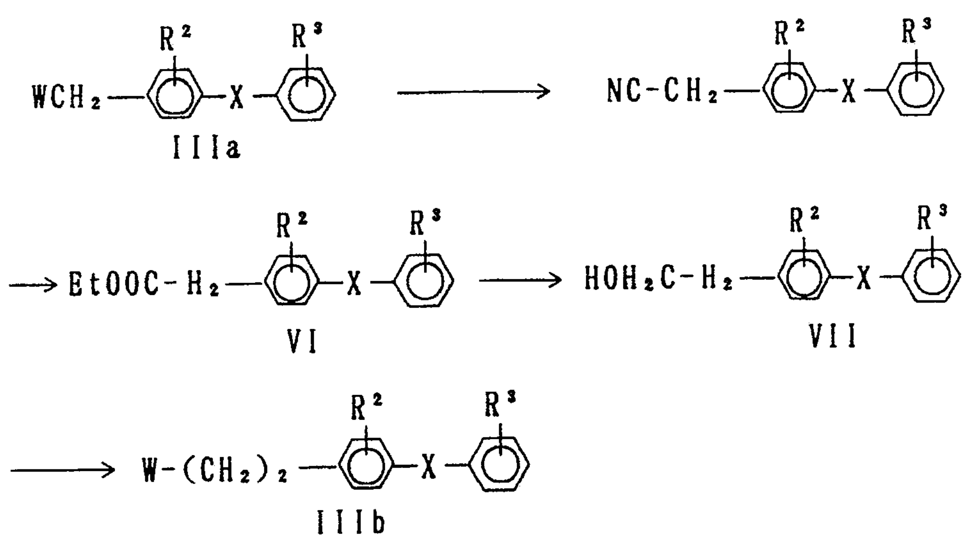

IIIa VI VII IIIb

wherein each group has the above-defined meaning.

The compounds (I) and salts thereof according to the present invention inhibit strongly vasoconstriction and hypertension derived by angiotensin II and therefore possess potent anti-hypertensive activity in animals, more specifically mammal animals (e.g. humans, dogs, rabbits, rats, etc.). Further, the compounds (I) and salts thereof according to the present invention are of quite low toxicity and useful in treating not only hypertension but also circulatory system diseases such as heart diseases, strokes and the like.

For therapeutic use, the compounds (I) and salts thereof can be administered as pharmaceutical compositions (e,g, powders, granules, tablets, pills, capsules, injections, solutions and the like) comprising at least one such compound alone or in admixture with pharmaceutically acceptable carriers, excipients and/or diluents. The pharmaceutical compositions can be formulated in accordance with a conventional method.

Specific dose levels for any particular patient will be employed depending upon a variety of factors including the activity of specific compounds employed, the age, body weight, general health, sex, diet, time of administration, route of administration, rate of excretion, drug combination, and the severity of the particular disease undergoing therapy. When used for treating adult essential hypertension, the active ingredient will be administered in an appropriate amount, for example, preferably selected from the range of about 10 mg to 100 mg a day orally and from the range of about 5 mg to 50 mg a day intravenously. The active ingredient will preferably be administered in equal doses two or three times a day.

The foregoing is merely illustrative of the invention and is not intended to limit the invention to the disclosed compounds. Variations and changes which are obvious to one skilled in the art are intended to be within the scope and nature of the invention.

## Example

The invention is further illustrated but in no way limited by the following working examples, reference examples and experimental examples.

In the specification of the present application, examples of the abbreviations used are given below. Me: Methyl, Et: Ethyl, Pr: propyl, Bu: Butyl, Tet: Tetrazolyl, DMF: Dimethylformamide.

## Pharmaceutical Examples

The compounds (I) of the present invention are employed for example, when used as agents for treating circulatory system diseases such as hypertension, heart diseases, strokes and the like, in the following formulations.

1. Capsule

| | | |
|---|---|---|
| (1) | 2-Butyl-1-[[2'-(1H-tetrazol-5-yl)-biphenyl-4-yl]methyl]imidazo-[4,5-b]pyridine | 10 mg |
| (2) | Lactose | 90 mg |
| (3) | Microcrystalline cellulose | 70 mg |
| (4) | Magnesium stearate | 10 mg |
| | One capsule | 180 mg |

The ingredients (1), (2), and (3) and a half of the ingredient (4) were blended together and granulated. To this mixture was added the remaining half of the ingredient (4) and distributed into gelatine capsules.

2. Tablet

| | | |
|---|---|---|
| (1) | 2-Butyl-1-[[2'-(1H-tetrazol-5-yl)-biphenyl-4-yl]methyl]imidazo-[4,5-b]pyridine | 10 mg |
| (2) | Lactose | 35 mg |
| (3) | Maize starch | 150 mg |
| (4) | Microcrystalline cellulose | 30 mg |
| (5) | Magnesium stearate | 5 mg |
| | One tablet | 230 mg |

Two third each of the ingredients (1), (2), (3) and (4) and a half of the ingredient (5) were blended together and granulated. To these granules were added the remaining ingredients (4) and (5) and then compressed to form tablets.

3. Injection

| | | |
|---|---|---|
| (1) | 2-Butyl-1-[[2'-(1H-tetrazol-5-yl)-biphenyl-4-yl]methyl]imidazo-[4,5-b]pyridine | 10 mg |
| (2) | Inositol | 100 mg |
| (3) | Benzyl alcohol | 20 mg |
| | One ampule | 130 mg |

The ingredients (1), (2) and (3) were dissolved in distilled water for injection to a total volume of two ml and distributed into ampules. Total processes were carried out under sterile conditions.

Reference Example 1

2-Butylimidazo[4,5-b]pyridine

2,3-Diaminopyridine (2.2 g), ethyl valeroimidate hydrochloride (8.3 g) and triethylamine (6.5 g) were dissolved in ethanol (20 ml) and heated under reflux for 12 hours with stirring. The mixture was concentrated to dryness and the resulting residue was extracted with ethyl acetate-water. The organic layer was washed with water, dried, and evaporated to dryness. The resulting residue was subjected to column chromatography on silica gel and the resulting crude crystalline was recrystallized from isopropyl ether to give the title compound (2.2 g, 63 %) as a colorless needle.
M.p. 104-105 °C.

Elemental Analysis for $C_{10}H_{13}N_3$

| | C (%) | H (%) | N (%) |
|---|---|---|---|
| Calcd: | C, 68.54; | H, 7.48; | N, 23.98 |
| Found: | C, 68.59; | H, 7.53; | N, 23.78. |

Reference Example 2

2-Butylimidazo[4,5-b]pyrazine

2,3-Diaminopyrazine (2.2 g), ethyl valeroimidate hydrochloride (6.6 g) and triethylamine (4.0 g) were dissolved in ethanol (30 ml) and heated under reflux for 10 hours with stirring. The mixture was concentrated and partitioned between brine and chloroform. The organic layer was washed with water, dried, and evaporated. The resulting crude crystals were recrystallized from ethyl acetate-methanol to give the title compound (2.1 g, 60 %) as a colorless plate.
M.p. 232-233 °C.

Elemental Analysis for $C_9H_{12}N_4$

| | C (%) | H (%) | N (%) |
|---|---|---|---|
| Calcd: | C, 61.34; | H, 6.86; | N, 31.79 |
| Found: | C, 61.38; | H, 6.93; | N, 31.64. |

Reference Example 3

2-Methylimidazo[4,5-b]pyridine

The compound was prepared according to the procedure for Reference Example 1.
$^1$H-NMR (200MHz, $CDCl_3$) δ : 2.73(3H, s), 7.24(1H, dd), 8.01 (1H, dd), 8.33(1H, dd).

Reference Example 4

2-Ethylimidazo[4,5-b]pyridine

The compound was prepared according to the procedure for Reference Example 1.
$^1$H-NMR (200MHz, $CDCl_3$) δ : 1.57(3H, t), 3.11 (2H, q), 7.25(1H, dd), 8.05(1H, dd), 8.34(1H, d).

Reference Example 5

2-Propylimidazo[4,5-b]pyridine

The compound was prepared according to the procedure for Reference Example 1.
$^1$H-NMR (200MHz, $CDCl_3$) δ: 1.10(3H, t), 1.93-2.12(2H, m), 3.05(2H, q), 7.25(1H, dd), 8.05(1H, dd), 8.33(1H, dd).

Reference Example 6

2-Propyl-1 -[[2'-(N-trityltetrazol-5-yl)-biphenyl-4-yl]methyl]imidazo[4,5-b]pyridine (A) and 2-propyl-3-[[2'-(N-trityltetrazol-5-yl)biphenyl-4-yl]-methyi]imidazo[4,5-b]pyridine (B)

To a cold stirred solution of 2-propylimidazo-[4,5-b]pyridine (0.81 g) in DMF (5 ml) was added sodium hydride (60% dispersion in mineral oil, 0.22 g) and the mixture was stirred at the same temperature for 15 minutes. To the mixture was added 4-[2'-(N-trityl-tetrazol-5-yl)phenyl]benzyl bromide (3.5 g) and the mixture was stirred at room temperature for 1.5 hours. The reaction mixture was diluted with water and extracted with ethyl acetate. The extract was washed with water, dried ($MgSO_4$), and evaporated to dryness to give a syrup. The resulting syrup was chromatographed on silica gel to give a brown syrup (A, 0.58 g, 18 %) and a pale brown syrup (B, 1.2 g, 38 %).

Compound A:

| | |
|---|---|
| $^1$H-NMR (200MHz, $CDCl_3$) $\delta$ : | 0.99(3H, t), 1.89(2H, m), 2.78(2H, q), 5.21 (2H, s), 6.77(2H, d), 6.89-7.00(8H, m), 7.09(2H, d), 7.17-7.36(10H, m), 7.43-7.49(2H, m), 7.91-7.95(1H, m), 8.46(1H, dd). |
| IR (neat) $cm^{-1}$: | 1600, 1500, 1490, 1465, 1440, 1400, 1350, 1320, 1275, 1205, 1180, 1020, 900, 870. |

Compound B:

| | |
|---|---|
| $^1$H-NMR (200MHz, $CDCl_3$) $\delta$ : | 0.93(3H, t), 1.79(2H, m), 2.68(2H, q), 5.40(2H, s), 6.90-6.95(8H, m), 7.07(2H, d), 7.18-7.34(1H, m), 7.41-7.46(2H, m), 7.88-7.96(1H, m), 7.99(1H, dd), 8.33(1H, dd). |
| IR (neat) $cm^{-1}$ : | 1600, 1500, 1450, 1425, 1400, 1355, 1280, 1225, 1190, 1030, 905, 880. |

Reference Example 7

2-Methyl-1-[[2'-(N-trityltetrazol-5-yl)-biphenyl-4-yl]methyl]imidazo[4,5-b]pyridine

The compound was prepared according to the procedure for Reference Example 6. A colorless syrup (10 %).

| | |
|---|---|
| $^1$H-NMR (200MHz, $CDCl_3$) $\delta$ : | 2.52(3H, s), 5.19(2H, s), 6.78(2H, d), 6.88-6.94(7H, m), 7.00(1H, dd), 7.10(2H, d), 7.17-7.36(10H, m), 7.44-7.49(2H, m), 7.93-7.98(1H, m), 8.48(1H, dd). |
| IR (neat) $cm^{-1}$ : | 1605, 1510, 1490, 1440, 1415, 1395, 1350, 1280, 1215, 1030, 1000, 875, 780, 760, 740. |

Reference Example 8

2-Methyl-3-[[2'-(N-trityltetrazol-5-yl)-biphenyl-4-yl]methyl]imidazo[4,5-b]pyridine

The compound was prepared according to the procedure for Reference Example 6.
A pale brown syrup (34 %).

| | |
|---|---|
| $^1$H-NMR (200MHz, $CDCl_3$) $\delta$ : | 2.41(3H, s), 5.37(2H, s), 6.88-6.95(8H, m), 7.08(2H, d), 7.18-7.35(1H, m), 7.43-7.49(2H, m), 7.91-7.95(1H, m), 7.98(1H, dd), 8.35(1H, dd). |
| IR (neat) $cm^{-1}$ : | 1600, 1515, 1460, 1445, 1425, 1390, 1280, 1215, 750. |

Reference Example 9

2-Ethyl-1-[[2'-(N-trityltetrazol-5-yl)-biphenyl-4-yl]methyl]imidazo[4,5-b]pyridine

The compound was prepared according to the procedure for Reference Example 6.
A brown syrup (5 %).

| | |
|---|---|
| $^1$H-NMR (200MHz, $CDCl_3$) $\delta$ : | 1.41(3H, t), 2.81(2H, q), 5.21(2H, s), 6.77(2H, d), 6.88-6.93(7H, m), 7.00(1H, dd), 7.09(2H, d), 7.18-7.36(1OH, m), 7.45-7.50(2H, m), 7.92-7.96(1H, m), 8.49(1H, dd). |
| IR (Nujol) $cm^{-1}$: | 1510, 1490, 1420, 1400, 1310, 1280, 1030, 1000, 880, 780, 760, 745, |

695.

Reference Example 10

2-Ethyl-3-[[2'-(N-trityltetrazol-5-yl)-biphenyl-4-yl]methyl]imidazo[4,5-b]pyridine

The compound was prepared according to the procedure for Reference Example 6.
A pale brown syrup (32 %).

| | |
|---|---|
| $^1$H-NMR (200MHz, $CDCl_3$) δ : | 1.32(3H, t), 2.71(2H, q), 5.39(2H, s), 6.89-6.95(8H, m), 7.07(2H, d), 7.19-7.35(1H, m), 7.43-7.49(2H, m), 7.90-7.94(1H, m), 8.04(1H, dd), 8.35(1H, dd). |
| IR (neat) $cm^{-1}$: | 1600, 1510, 1495, 1440, 1420, 1400, 1280, 1230, 1025, 900, 880, 795, 750, 695. |

Reference Example 11

2-Butylimidazo[4,5-c]pyridine

The compound was prepared according to the procedure for Reference Example 1.
A pale brown oil.

| | |
|---|---|
| $^1$H-NMR (200MHz, $CDCl_3$) δ : | 0.91(3H, t), 1.33-1.52(2H, m), 1.82-1.97(2H, m), 3.02(2H, t), 7.50(1H, d), 8.38(1H, d), 8.38(1H, d), 8.94(1H, s). |
| IR (neat) $cm^{-1}$ : | 1620, 1590, 1535, 1460, 1420, 1280, 1025, 810, 755. |

Reference Example 12

2-Butyl-3-[[2'-(N-trityltetrazol-5-yl)-biphenyl]methyl]imidazo[4,5-c]pyridine

The compound was prepared according to the procedure for Reference Example 6.
A pale brown oil (6.5 %).

| | |
|---|---|
| $^1$H-NMR (200MHz, $CDCl_3$) δ : | 0.92(3H, t), 1.29-1.48(2H, m), 1.75-1.90(2H, m), 2.78(2H, t), 5.27(2H, s), 6.82(2H, d), 6.90-6.94(8H, m), 7.11(2H, d), 7.19-7.36(8H, m), 7.45-7.51-(2H, m), 7.68(1H, dd), 7.93-7.98(1H, m), 8.44(1H, d), 8.56(1H, d). |

Reference Example 13

2-Butyl-1-[[2'-(N-trityltetrazol-5-yl)-biphenyl]methyl]imidazo[4,5-c]pyridine

The compound was prepared according to the procedure for Reference Example 6.
A pale brown oil (8.8 %).

| | |
|---|---|
| $^1$H-NMR (200MHz, $CDCl_3$) δ : | 0.92(3H, t), 1.29-1.49(2H, m), 1.75-1.91(2H, m), 2.78(2H, t), 5.21(2H, s), 6.78(2H, d), 6.90-7.01(7H, m), 7.11(2H, d), 7.19-7.37(10H, m), 7.44-7.50(2H, m), 7.92-7.97(1H, m), 8.27(1H, d), 9.09(1H, s). |

Reference Example 14

2-Butyl-6-methylimidazo[4,5-b]pyridine

The compound was prepared according to the procedure for Reference Example 1.
Colorless prisms (41 %).
M.p. 156-157° C.

| | |
|---|---|
| $^1$H-NMR (200MHz, $CDCl_3$) δ : | 1.00(3H, t), 1.40-1.59(2H, m), 1.87-2.02(2H, m), 2.49(3H, s), 3.03(2H, t), 7.82(1H, s), 8.14(1H, s). |
| IR (KBr) $cm^{-1}$: | 3300-2200, 1620, 1545, 1480, 1440, 1410, 1395, 1375, 1310, 1275, 1255, 1150, 990, 975, 880, 860, 765, 735. |

Reference Example 15

2-Butyl-6-methyl-3-[[2'-(N-trityltetrazol-5-yl)biphenyl-4-yl]methyl]imidazo[5,4-b]pyridine

The compound was prepared according to the procedure for Reference Example 6.
A colorless syrup (26 %).

$^1$H-NMR (200MHz, $CDCl_3$) δ: 0.88(3H, t), 1.24-1.39(2H, m), 1.67-1.82(2H, m), 2.48(3H, s), 2.69(2H, t), 5.38(2H, s), 6.88-6.96(8H, m), 7.06(2H, d), 7.20-7.36(10H, m), 7.43-7.49-(2H, m), 7.81(1H, d), 7.89-7.93(1H, m), 8.18(1H, d).

IR (neat) $cm^{-1}$ : 1510, 1440, 1420, 1390, 1365, 1355, 1235, 1040, 1020, 1000, 880, 745, 695.

Reference Example 16

2-Butyl-6-methyl-1-[[2'-(N-trityltetrazol-5-yl)biphenyl-4-yl]methyl]imidazo[4,5-b]pyridine

The compound was prepared according to the procedure for Reference Example 6.
Colorless prisms (12 %).
M.p. 179-180° C (dec.).

$^1$H-NMR (200MHz, $CDCl_3$) δ : 0.90(3H, t), 1.30-1.48(2H, m), 1.76-1.91(2H, m), 2.28(3H, s), 2.78(2H, t), 5.18(2H, s), 6.76(2H, d), 6.89-6.96(6H, m), 7.08-7.13(3H, m), 7.18-7.38-(10H, m), 7.45-7.51 (2H, m), 7.91-7.95(1H, m), 8.33(1H, d).

IR (KBr) $cm^{-1}$: 1510, 1495, 1460, 1440, 1400, 1275, 1030, 1000, 880, 870, 750, 740, 695.

Reference Example 17

2-Amino-6-dimethylamino-3-nitropyridine

A mixture of 2-amino-6-chloro-3-nitropyridine (1.5 g) and 50 % aqueous dimethylamine solution (3.9 g) in ethanol (30 ml) was refluxed for 13 hours. The reaction mixture was cooled to give a yellow crystalline product, which was collected by filtration and washed with ethanol to give yellow plates (1.08 g, 69 %).
M.p. 162-163° C.

$^1$H-NMR (200MHz, $CDCl_3$) δ : 3.16(6H, s), 6.02(1H, d), 8.17(1H, d).

Reference Example 18

5-Dimethylamino-2-propylimidazo[4,5-b]pyridine

To a solution of 2-amino-6-dimethylamino-3-nitropyridine (1.07 g) and conc. $NH_4OH$ (4.2 ml) in tetrahydrofuran (20 ml) and water (17 ml) was added a solution of $Na_2S_2O_4$(6.73 g) in water (25 ml). The mixture was allowed to stir at room temperature for 4 hours and then stirred at 50° C for 14 hours. The reaction mixture was extracted with ethyl acetate. The extract was washed with water, dried, and evaporated to dryness. The resulting product was mixed with polyphosphoric acid (10 g) and n-butyric acid (1 ml) and the mixture was heated at 130° C for 4 hours. The reaction mixture was dissolved in water and the aqueous solution was made basic by addition of $NaHCO_3$ to separate an oily product. The oil was dissolved in ethyl acetate and the solution was washed with water, dried, and evaporated to dryness. The resulting product was purified by column chromatography on silica gel to to give a brown powder (0.4 g, 33 %).
M.p. 159-160° C.

$^1$H-NMR (200MHz, $CDCl_3$) δ : 1.01(3H, t), 1.74-1.92(2H, m), 2.82(2H, t), 3.11(6H, s), 6.46(1H, d), 7.74-(1H, d).

Reference Example 19

2-Amino-3-nitro-6-propylthiopyridine

A mixture of 2-amino-6-chloro-3-nitropyridine (1.5 g) and propylmercaptan (1.57 ml) and triethylamine (1.75 g) in 1,2-dichloroethane (30 ml) was heated at reflux for 19 hours. The reaction mixture was evaporated to dryness to give a solid.
Recrystallization from aqueous ethanol gave yellow prisms (1.14 g, 62 %).

M.p. 72-73 °C.
$^1$H-NMR (200MHz, $CDCl_3$) δ : 1.05(3H, t), 1.66-1.83(2H, m), 3.13(2H, t), 6.55(1H, d), 8.16(1H, d).

Reference Example 20

2-Propyl-5-propylthioimidazo[4,5-b]pyridine

The compound was prepared according to the procedure for Reference Example 18.
A brown powder (62 %).
M.p. 82-83 °C.
$^1$H-NMR (200MHz, $CDCl_3$) δ: 1.04(3H, t), 1.05(3H, t), 1.68-1.85(2H, m), 1.81-2.01(2H, m), 3.00(2H, t), 3.14(2H, t), 7.13(1H, d), 7.81(1H, d).

Reference Example 21

5-Chloro-2-propylimidazo[4,5-b]pyridine

The compound was prepared according to the procedure for Reference Example 18.
A pale brown powder (22 %).
M.p. 164-166 °C.
$^1$H-NMR (200MHz, $CDCl_3$) δ : 1.06(3H, t), 1.87-2.05(2H, m), 3.06(2H, t), 7.26(1H, d), 7.98(1H, d).

Reference Example 22

2-Propyl-5-propylthio-3-[[2'-(N-trityltetrazol-5-yl)biphenyl-4-yl]methyl]imidazo[4,5-b]-pyridine (A) and 2-propyl-5-propylthio-1-[[2'-(N-trityltetrazol-5-yl)biphenyl-4-yl]methyl]imidazo[4,5-b]-pyridine (B)

To a solution of 2-propyl-5-propylthioimidazo-[4,5-b]pyridine (0.77 g) in DMF (15 ml) was added sodium hydride (60% dispersion in mineral oil, 0.16 g) under cooling in an ice bath and the mixture was allowed to stir at room temperature for 30 minutes. To the mixture was added [2'-(N-trityltetrazol-5-yl)biphenyl-4-yl]-methyl bromide (2.19 g) and the mixture was stirred at the same temperature for 3 hours. The reaction mixture was concentrated to dryness and the resulting residue was dissolved in ethyl acetate. The solution was washed with water, dried, and evaporated to dryness to give a residue, which was chromatographed on silica gel to give a pale powder (A, 1.6 g, 67 %) and a yellow syrup (B, 0.45 g, 19 %).

Compound A:
M.p. 102-106 °C.

$^1$H-NMR (200MHz, $CDCl_3$) δ : 0.94(3H, t), 0.96(3H, t), 1.58-1.89(4H, m), 2.67(2H, t), 3.14(2H, t), 5.33-(2H, s), 6.89-6.99(8H, m), 7.06-7.36(13H, m), 7.43-7.50(2H, m), 7.83-(1H, d), 7.89-7.94(1H, m).

Compound B:

$^1$H-NMR (200MHz, $CDCl_3$) δ : 0.98(3H, t), 1.05(3H, t), 1.70-2.00(4H, m), 2.75(2H, t), 3.32(2H, t), 5.16-(2H, s), 6.75(2H, d), 6.83-6.96(7H, m), 7.04-7.11(3H, m), 7.17-7.37(10H, m). 7.42-7.52(2H, m), 7.91-7.97(1H, m).

Reference Example 23

5-Chloro-2-propyl-3-[[2'-(N-trityltetrazol-5-yl)biphenyl-4-yl]methyl]imidazo[4,5-b]pyridine

The compound was prepared according to the procedure for Reference Example 22.
A pale yellow powder (52 %).
$^1$H-NMR (200MHz, $CDCl_3$) δ : 0.93(3H, t), 1.71-1.89(2H, m), 2.66(2H, t), 5.33(2H, s), 6.86-6.97(8H, m), 7.08(2H, d), 7.21-7.52(13H, m), 7.92-7.98(1H, m), 7.96(1H, d).

Reference Example 24

5-Chloro-2-propyl-1-[[2'-(N-trityltetrazol-5-yl)biphenyl-4-yl]methyl]imidazo[4,5-b]pyridine

The compound was prepared according to the procedure for Reference Example 22.

A pale yellow powder (30 %).

$^1H$-NMR (200MHz, $CDCl_3$) δ : 0.99(3H, t), 1.80-1.99(2H, m), 2.79(2H, t), 5.21(2H, s), 6.77(2H, d), 6.89-6.98(6H, m), 7.09-7.38(14H, m), 7.43-7.53 (2H, m), 7.91-7.98(1H, m).

Reference Example 25

5-Dimethylamino-2-propyl-3-[[2'-(N-trityltetrazol-5-yl)biphenyl-4-yl]methyl]imidazo[4,5-b]-pyridine

The compound was prepared according to the procedure for Reference Example 22.

A pale brown powder (28 %).

$^1H$-NMR (200MHz, $CDCl_3$) δ: 0.92(3H, t), 1.68-1.85(2H, m), 2.60(2H, t), 3.08(6H, s), 5.25(2H, s), 6.49-(1H, d), 7.80(1H, d), 6.88-7.51(22H, m), 7.88-7.93(1H, m).

Reference Example 26

5-Methoxy-2-propylimidazo[4,5-b]pyridine

The compound was prepared according to the procedure for Reference Example 5.

A brown syrup (95 %).

$^1H$-NMR (200MHz, $CDCl_3$) δ : 1.02(3H, t), 1.89(2H, m), 2.92(2H, t), 3.96(3H, s), 6.45(1H, d), 7.81(1H, d).

IR (neat) $cm^{-1}$: 1610, 1590, 1485, 1440, 1405, 1325, 1270, 1245, 1220, 1200, 1030, 810, 755.

Reference Example 27

5-Methoxy-2-propyl-1-[[2'-(N-trityltetrazol-5-yl)biphenyl-4-yl]methyl]imidazo[4,5-b]pyridine

The compound was prepared according to the procedure for Reference Example 22.

Colorless amorphus powders (33 %).

M.p. 95-96 °C.

$^1H$-NMR (200MHz, $CDCl_3$) δ: 0.98(3H, t), 1.91(2H, m), 2.72(2H, q), 4.03(3H, s), 5.17(2H, s), 6.48(1H, d), 6.76(2H, d), 6.89-6.95(6H, m), 7.09(2H, d), 7.17-7.37(11H, m), 7.45-7.51(2H, m), 7.92-7.96(1H, m).

IR (KBr) $cm^{-1}$: 1580, 1500, 1475, 1440, 1400, 1225, 1200, 1025, 745, 695.

Reference Example 28

5-Methoxy-2-propyl-3-[[2'-(N-trityltetrazol-5-yl)biphenyl-4-yl]methyl]imidazo[4,5-b]pyridine

The compound was prepared according to the procedure for Reference Example 22.

Colorless needles (42 %).

M.p. 172-173 °C (dec.).

$^1H$-NMR (200MHz, $CDCl_3$) δ: 0.94(3H, t), 1.79(2H, m), 2.64(2H, q), 3.89(3H, s), 5.31(2H, s), 6.68(1H, d), 6.90-6.98(8H, m), 7.09(2H, d), 7.18-7.37 (1H, m), 7.44-7.50(2H, m), 7.89-7.93(2H, m).

IR (KBr) $cm^{-1}$: 1595, 1490, 1440, 1420, 1400, 1340, 1245, 1025, 810, 745, 695, 690.

Reference Example 29

6-Bromo-2-butyl-5-methylimidazo[4,5-b]-pyrimidine

The compound was prepared according to the procedure for Reference Example 1.

Colorless needles (35 %).

M.p. 141-143 °C.

$^{1}$H-NMR (200MHz, $CDCl_3$) δ: 0.96(3H, t), 1.36-1.54(2H, m), 1 78-1.93(2H, m), 2.79(3H, s), 2.97(2H, t), 8.16(1H, s), 11.53(1H,brs).
IR (KBr) $cm^{-1}$: 1610, 1570, 1530, 1460, 1430, 1400, 1260, 1090, 980, 870.

Reference Example 30

6-Bromo-2-butyl-5-methyl-3-[[2'-(N-trityltetrazol-5-yl)biphenyl-4-yl]methyl]imidazo[5,4-b]-pyridine

The compound was prepared according to the procedure for Reference Example 22.
A colorless syrup (56 %).
$^{1}$H-NMR (200MHz, $CDCl_3$) δ : 0.87(3H, t), 1.22-1.43(2H, m), 1.65-1.80(2H, m), 2.68(2H, t), 2.70(3H, s), 5.34(2H, s), 6.84-6.94(8H, m), 7.06(2H, d), 7.19-7.36(10H, m), 7.41-7.52(2H, m), 7.90-7.94 (1H, m), 8.13(1H, s).
IR (neat) $cm^{-1}$: 1495, 1450, 1400, 1360, 1240, 1040, 750, 700.

Reference Example 31

6-Bromo-2-butyl-5-methyl-1-[[2'-(N-trityltetrazol-5-yl)biphenyl-4-yl]methyl]imidazo[5,4-b]-pyridine

The compound was prepared according to the procedure for Reference Example 22.
Colorless powders.
$^{1}$H-NMR (200MHz, $CDCl_3$) δ : 0.90(3H, t), 1.30-1.47(2H, m), 1.76-1.91(2H, m), 2.74(2H, t), 2.75(3H, s), 5.13(2H, s), 6.73(2H, d), 6.88-6.93(6H, m), 7.10(2H, d), 7.18-7.37(10H, m), 7.45-7.49(2H, m), 7.51(1H, s), 7.93-7.98(1H, m).
IR (KBr) $cm^{-1}$: 1445, 1400, 750, 700.

Reference Example 32

2-Propylimidazo[4,5-b]pyridine 4N-oxide

A mixture of 2-propylimidazo[4,5-b]pyridine (2.5 g) in hydrogen peroxide (30 %, 3 ml) and acetic acid (1.4 ml) was stirred at 110-120°C for 9 hours. The reaction mixture was evaporated to dryness and the residue was purified by column chromatography on silica gel. The resulting crystals were recrystallized from methanol-ethyl acetate to give colorless prisms (1.7 g, 61 %).
M.p. 193-194°C.

Elemental Analysis for $C_9H_{11}N_3O$

| | C (%) | H (%) | N (%) |
|---|---|---|---|
| Calcd: | C, 61.00; | H, 6.26; | N, 23.71 |
| Found: | C, 61.05; | H, 6.23; | N, 23.69. |

$^{1}$H-NMR (200MHz, DMSO-$d_6$) δ : 0.94(3H, t), 1.80(2H, m), 2.83(2H, t), 7.14(1H, dd), 7.50(1H, d), 8.12-(1H, d).
IR (KBr) $cm^{-1}$: 3150-2200, 1585, 1525, 1460, 1440, 1420, 1270, 1235, 1205, 1165, 1080, 990, 780, 740.

Reference Example 33

2-Propyl-1-[[2'-(N-trityltetrazol-5-yl)-biphenyl-4-yl]methyl]imidazo[4,5-b]pyridine 4N-oxide

The compound was prepared according to the procedure for Reference Example 22.
A pale yellow oil (20 %).
$^{1}$H-NMR (200MHz, $CDCl_3$) δ : 0.98(3H, t), 1.91(2H, m), 2.81(2H, q), 5.23(2H, s), 6.78-6.95(10H, m),

7.12-7.38(12H, m), 7.46-7.54(2H, m), 7.92-7.97 (1H, m), 8.19(1H, dd).

IR (KBr) $cm^{-1}$: 1580, 1500, 1460, 1440, 1410, 1375, 1325, 1250, 1220, 1060, 1010, 1000, 900, 880, 760.

Reference Example 34

2-Propyl-3-[[2'-(N-trityltetrazol-5-yl)-biphenyl-4-yl]methyl]imidazo[4,5-b]pyridine 4N-oxide

The compound was prepared according to the procedure for Reference Example 22.
A pale yellow oil (52 %).

$^1$H-NMR (200MHz, $CDCl_3$) δ : 1.09(3H, t), 2.02(2H, m), 3.08(2H, t), 5.59(2H, s), 6.58(1H, dd), 6.94-7.52(23H, m), 7.86(1H, d), 7.90-7.95 (1H, m).

IR (neat) $cm^{-1}$ : 1615, 1490, 1445, 1375, 1275, 1250, 1215, 1190, 880, 850, 750, 695.

Reference Example 35

2-Cyano-2-propylimidazo[4,5-b]pyridine

A mixture of 2-propylimidazo[4,5-b]pyridine 4N-oxide (2.7 g), trimethylsilylcyanide (6 g) and triethylamine (3 g) in acetonitrile (20 ml) was heated at reflux for 15 hours. The reaction mixture was evaporated to dryness and the resulting syrup was purified by column chromatography on silica gel to give a crystalline product. Recrystallization from ethyl acetate gave colorless prisms (2.2 g, 79 %).
M.p. 166-167 °C.

$^1$H-NMR (200MHz, $CDCl_3$) δ : 1.10(3H, t), 2.00(2H, m), 3.16(2H, t), 7.69(1H, d), 8.12(1H, d).

IR (KBr) $cm^{-1}$: 2225, 1620, 1540, 1430, 1410, 1380, 1010, 850, 795, 750.

Reference Example 36

5-Cyano-2-propyl-1-[[2'-(N-trityltetrazol-5-yl)biphenyl-4-yl]methyl]imidazo[4,5-b]pyridine

The compound was prepared according to the procedure for Reference Example 6.
Colorless needles (25 %).
M.p. 164-165 °C (dec.).

$^1$H-NMR (200MHz, $CDCl_3$) δ: 0.97(3H, t), 1.83(2H, m), 2.75(2H, t), 5.37(2H, s), 6.89-6.93(8H, m), 7.09-(2H, d), 7.21-7.40(10H, m), 7.44-7.50 (2H, m), 7.65(1H, d), 7.93-7.97(1H, m), 8.08(1H, d).

IR (KBr) $cm^{-1}$: 2220, 1600, 1500, 1470, 1445, 1420, 1400, 1310, 1270, 880, 845, 810, 755, 745, 700.

Reference Example 37

5-Cyano-2-propyl-3-[[2'-(N-trityltetrazol-5-yl)biphenyl-4-yl]methyl]imidazo[4,5-b]pyridine

The compound was prepared according to the procedure for Reference Example 6.
Colorless crystals (40 %).
M.p. 188-189 °C (dec.).

$^1$H-NMR (200MHz, $CDCl_3$) δ : 0.97(3H, t), 1.83(2H, m), 2.75(2H, t), 5.37(2H, s), 6.89-6.93(8H, m), 7.09(2H, d), 7.21-7.40(10H, m), 7.44-7.50 (2H, m), 7.65(1H, d), 7.93-7.97(1H, m), 8.08(1H, d).

IR (KBr) $cm^{-1}$: 2220, 1600, 1500, 1470, 1445, 1420, 1400, 1310, 1270, 880, 845, 810, 755, 745, 700.

Reference Example 38

2-Propylimidazo[4,5-c]pyridine

The compound was prepared by the procedure for Reference Example 11.
A pale brown oil (91 %).

$^1$H-NMR (200MHz, $CDCl_3$) δ : 1.01(3H, t), 1.92(2H, m), 2.97(2H, t), 7.48(1H, dd), 8.34(1H, d), 8.90(1H, d).
IR (neat) $cm^{-1}$ : 1620, 1590, 1440, 1420, 1280, 1200, 1030, 810, 750.

Reference Example 39

2-Propylimidazo[4,5-c]pyridine 5N-oxide

The compound was prepared by the procedure for Reference Example 32.
A brown oil (54 %).
$^1$H-NMR (200MHz, $CDCl_3$) δ : 0.99(3N, t), 1.88(2H, m), 2.92(2H, t), 7.50(1H, d), 8.10(1H, dd), 8.68(1H, d).
IR (neat) $cm^{-1}$ : 1535, 1450, 1265, 1130, 990, 860, 805, 790, 770, 725.

Reference Example 40

4-Cyano-2-propylimidazo[4,5-c]pyridine

The compound was prepared by the procedure for Reference Example 35.
Colorless prisms (61 %).
M.p. 178-179 ° C.
$^1$H-NMR (200MHz, $CDCl_3$) δ : 1.07(3H, t), 1.96(2H, m), 3.04(2H, t), 7.71(1H, d), 8.50(1H, d).
IR (neat) $cm^{-1}$ : 3200-2250, 2220, 1615, 1575, 1530, 1420, 1280, 1260, 1215, 1090, 1070, 1005, 835.

Reference Example 41

4-Cyano-2-propyl-3-[[2'-(N-trityltetrazol-5-yl)biphenyl-4-yl]methyl]imidazo[4,5-c]pyridine

The compound was prepared by the procedure for Reference Example 22.
Colorless powders (4 %).
M.p. 95-97 ° C.
$^1$H-NMR (200MHz, $CDCl_3$) δ: 0.96(3N, t), 1.86(2H, m), 2.73(2H, t), 5.64(2H, s), 6.81(2H, d), 6.92-7.98-(6H, m), 7.13(2H, d), 7.20-7.37(10H, m), 7.44-7.50(2H, m), 7.89(1H, d), 7.91-7.95 (1H, m), 8.53(1H, d).
IR (KBr) $cm^{-1}$: 2220, 1600, 1500, 1455, 1440, 1430, 1400, 1275, 1195, 1020, 1000, 880, 820, 760, 740, 695.

Reference Example 42

4-Cyano-2-propyl-1-[[2'-(N-trityltetrazol-5-yl)biphenyl-4-yl]methyl]imidazo[4,5-c]pyridine

The compound was prepared by the procedure for Reference Example 22.
Colorless prisms (70 %).
M.p. 121-124 ° C.
$^1$H-NMR (200MHz, $CDCl_3$) δ: 1.01(3H, t), 1.88(2H, m), 2.84(2H, t), 5.25(2H, s), 6.77(2H, d), 6.90-6.94-(6H, m), 7.10-7.37(13H, m), 7.46-7.52(2H, m), 7.92-7.97(1H, m), 8.29-(1H, d).
IR (KBr) $cm^{-1}$: 2220, 1600, 1580, 1495, 1440, 1395, 1270, 1235, 1225, 1200, 1030, 830, 745, 695.

Working Example 1

2-Butyl-3-[(2'-cyanobiphenyl-4-yl)methyl]-imidazo[4,5-b]pyridine

To a solution of 2-butylimidazo[4,5-b]pyridine (1.05 g) in DMF (10 ml) was added sodium hydride (60% dispersion in mineral oil, 0.26 g) under ice-cooling and stirred for 20 minutes. To the mixture was added a solution of 4-(2-cyanophenyl)benzyl chloride (1:37 g) and stirred at room temperature for 2.5 hours. To the

reaction mixture was added water and ethyl acetate and it was partitioned. The organic phase was washed with water, dried, and evaporated to yield the residue (1.25 g, 57 %) as a brown oil.

$^1$H-NMR (200MHz, $CDCl_3$) δ : 0.92(3H, t), 1.34-1.52(2H, m), 1.75-1.91(2H, m), 2.85(2H, t), 5.57(2H, s), 7.22(1H, d), 7.26(2H, d), 7.39-7.53(5H, m), 7.59-7.67(1H, m), 7.73-7.78-(1H, m), 8.03(1H, dd), 8.36(1H, dd).

IR (neat) $cm^{-1}$ : 2220, 1600, 1510, 1480, 1450, 1425, 1400, 1280, 1240, 760.

Working Example 2

2-Butyl-1-[(2'-cyanobiphenyl-4-yl)methyl]-imidazo[4,5-b]pyridine

The product obtained in the same manner as in Working Example 1 was purified by column chromatography on silica gel to yield the title compound (0.3 g, 14 %) as a brown oil.

$^1$H-NMR (200MHz, $CDCl_3$) δ : 0.94(3H, t), 1.37-1.56(2H, m), 1.82-1.97(2H, m), 2.93(2H, t), 5.43(2H, s), 7.13(1H, d), 7.15(2H, d), 7.42-7.54(5H, m), 7.61-7.69(1H, m), 7.77(1H, dd), 8.53(1H, dd).

IR (neat) $cm^{-1}$ : 2230, 1610, 1515, 1485, 1420, 1280, 760.

Working Example 3

2-Butyl-3-[2'-[(1H-tetrazol-5-yl)biphenyl-4-yl]methyl]imidazo[4,5-b]pyridine

2-Butyl-3-[(2-cyanobiphenyl-4-yl)methyl]-imidazo[4,5-b]pyridine (1.25 g), sodium azide (3.3 g) and ammonium chloride (2.7 g) in DMF (12 ml) were stirred at 115°C for 5 days. To the reaction mixture was added water and ethyl acetate and it was partitioned. The organic phase was washed with water, dried, and concentrated. The resulting residue was subjected to column chromatography on silica gel and the resulting crude crystals were recrystallized from ethyl acetate-methanol to give the title compound (0.82 g, 59 %) as a colorless prism.

M.p. 197-198°C.

Elemental Analysis for $C_{24}H_{23}N_7$

| | C (%) | H (%) | N (%) |
|---|---|---|---|
| Calcd: | C, 70.40; | H, 5.66; | N, 23.94 |
| Found: | C, 70.42; | H, 5.73; | N, 23.64. |

$^1$H-NMR (200MHz, DMSO-$d_6$) δ : 0.86(3H, t), 1.25-1.44(2H, m), 1.61-1.76(2H, m), 2.82(2H, t), 5.52(2H, s), 7.05(2H, d), 7.12(2H, d), 7.26(1H, dd), 7.48-7.71(4H, m), 8.00-(1H, dd), 8.30(1H, dd).

IR (KBr) $cm^{-1}$ : 1600, 1510, 1465, 1410, 1280, 780, 760.

Working Example 4

2-Butyl-1-[2'-[(1H-tetrazol-5-yl)biphenyl-4-yl]methylimidazo[4,5-b]pyridine

2-Butyl-1-[(2'-cyanobiphenyl-4-yl)methyl]-imidazo[4,5-b]pyridine (0.3 g), sodium azide (0.78 g) and ammonium chloride (0.64 g) in DMF (4 ml) were stirred at 115°C for 7 days. To the reaction mixture was added water, it was neutralized with 1N-hydrochloric acid, and extracted with ethyl acetate. The organic phase was washed with water, dried, and evaporated. The resulting residue was subjected to column chromatography on silica gel and the resulting crude crystalline was recrystallized from ethyl acetate-methanol to give the title compound (77 mg, 22 %) as a colorless crystalline.

M.p. 137-139°C.

Elemental Analysis for $C_{24}H_{23}N_7 \cdot 0.4\ H_2O$

| | C (%) | H (%) | N (%) |
|---|---|---|---|
| Calcd: | C, 69.18; | H, 5.76; | N, 23.53 |
| Found: | C, 69.42; | H, 5.93; | N, 23.33. |

$^1$H-NMR (200MHz, $CDCl_3$) δ : 0.85(3H, t), 1.22-1.42(2H, m), 1.59-1.74(2H, m), 2.66(2H, t), 5.26(2H, s), 6.69(2H, d), 6.97(2H, d), 7.02(1H, dd), 7.31-7.36(1H, m), 7.41(1H, dd), 7.49-7.60(2H, m), 7.90-7.94(1H, m), 8.19(1H, dd).

IR (KBr) $cm^{-1}$ : 1610, 1510, 1480, 1450, 1415, 1280, 780, 760.

Working Example 5

2-Butyl-1-[(2'-cyanobiphenyl-4-yl)methyl]-imidazo[4,5-b]pyrazine

To a solution of 2-butylimidazo[4,5-b]pyrazine (0.35 g) in DMF (5 ml) was added sodium hydride (60% dispersion in mineral oil, 88 mg) under ice-cooling and stirred for 15 minutes. To the mixture was added a solution of 4-(2-cyanophenyl)benzyl chloride (0.46 g) and stirred for 2 hours. To the reaction mixture was added water and was extracted with ethyl acetate. The organic phase was washed with water, dried, and evaporated. The resulting product was purified by column chromatography on silica gel to yield the title compound (0.36 g, 49 %) as a pale brown oil.

$^1$H-NMR (200MHz, $CDCl_3$) δ : 0.90(3H, t), 1.28-1.43(2H, m), 1.73-1.89(2H, m), 2.79(2H, t), 5.38(2H, s), 7.26(2H, d), 7.43-7.55(4H, m), 7.60-7.75(2H, m), 8.26(1H, d), 8.50(1H, d).

Working Example 6

2-Butyl-1-[[2'-(1H-tetrazol-5-yl)biphenyl-4-yl]methyl]imidazo[4,5-b]pyrazine

2-Butyl-1-[(2'-cyanobiphenyl-4-yl)methyl]-imidazo[4,5-b]pyrazine (0.36 g), sodium azide (0.96 g) and ammonium chloride (0.79 g) in DMF (5 ml) were stirred at 115 °C for 5 days. To the reaction mixture was added water and ethyl acetate and it was partitioned. The organic phase was washed with water, dried, and concentrated. The resulting product was subjected to column chromatography on silica gel to give the title compound (0.12 g, 29 %) as a colorless crystalline powder.

M.p. 102-104 °C.

Elemental Analysis for $C_{23}H_{22}N_8 \cdot 0.4\ H_2O$

| | C (%) | H (%) | N (%) |
|---|---|---|---|
| Calcd: | C, 66.14; | H, 5.50; | N, 26.83 |
| Found: | C, 66.44; | H, 5.47; | N, 26.56. |

$^1$H-NMR (200MHz, $CDCl_3$) δ : 0.87(3H, t), 1.28-1.46(2H, m), 1.67-1.82(2H, m), 2.80(2H, t), 5.45(2H, s), 7.03(2H, d), 7.10(2H, d), 7.36(1H, dd), 7.47-7.62(2H, m), 7.97(1H, dd), 8.25(1H, d), 8.38(1H, d).

IR (KBr) $cm^{-1}$: 1570, 1495, 1450, 1420, 1400, 1360, 1200, 845, 775, 755.

Working Example 7

2-Methyl-3-[[2'-(1H-tetrazol-5-yl)biphenyl-4-yl]methyl]imidazo[4,5-b]pyridine

A mixture of 2-methyl-3-[[2'-(trityltetrazol-5-yl)biphenyl-4-yl]methyl]imidazo[4,5-b]pyridine (0.89 g) in methanol (15 ml) containing 1 N HCl (3 ml) was allowed to stir at room temperature for 3 hours. The reaction mixture was neutralized by addition of 1N NaOH and then concentrated to dryness to give a syrup. The syrup was dissolved in chloroform and the solution was washed with water, dried ($MgSO_4$), and evaporated to dryness. The resulting syrup was purified by column chromatography on silica gel to give a crystalline product. Recrystallization from methanol-ethyl acetate gave colorless prisms (0.29 g, 53 %).
M.p. 251-254 °C (dec.).

Elemental Analysis for $C_{21}H_{17}N_7$

| | C (%) | H (%) | N (%) |
|---|---|---|---|
| Calcd: | C, 68.65; | H, 4.66; | N, 26.69 |
| Found: | C, 68.58; | H, 4.75; | N, 26.57. |

$^1$H-NMR (200MHz, DMSO-$d_6$) δ : 2.51(3H, s), 5.51(2H, s), 7.05(2H, d), 7.14(2H, d), 7.26(1H, dd), 7.50-7.71(4H, m), 7.98(1H, dd), 8.30(1H, dd).
IR (KBr) $cm^{-1}$: 1600, 1465, 1420, 1400, 1350, 1290, 1240, 1040, 985, 960, 855, 800, 780, 755.

Working Example 8

2-Methyl-1-[[2'-(1H-tetrazol-5-yl)biphenyl-4-yl]methyl]imidazo[4,5-b]pyridine

The compound was prepared according to the procedure for Working Example 7.
Colorless needles (41 %).
M.p. 180-182 °C.

Elemental Analysis for $C_{21}H_{17}N_7 \cdot 1.1\ H_2O$

| | C (%) | H (%) | N (%) |
|---|---|---|---|
| Calcd: | C, 65.14; | H, 5.00; | N, 25.32 |
| Found: | C, 65.31; | H, 4.94; | N, 25.03. |

$^1$H-NMR (200MHz, DMSO-$d_6$) δ : 2.56(3H, s), 5.52(2H, s), 7.04-7.13(4H, m), 7.19(1H, dd), 7.49-7.70-(4H, m), 7.93(1H, dd), 8.34(1H, dd).
IR (KBr) $cm^{-1}$: 1600, 1510, 1415, 1280, 775, 755.

Working Example 9

2-Ethyl-3-[[2'-(1H-tetrazol-5-yl)biphenyl-4-yl]methyl]imidazo[4,5-b]pyridine

The compound was prepared according to the procedure for Working Example 7.
Colorless needles (66 %).
M.p. 149-151 °C (dec.).

Elemental Analysis for $C_{22}H_{19}N_7 \cdot 1/2$ EtOAc

| | C (%) | H (%) | N (%) |
|---|---|---|---|
| Calcd: | C, 67.75; | H, 5.45; | N, 23.04 |
| Found: | C, 67.52; | H, 5.37; | N, 23.13. |

$^1$H-NMR (200MHz, DMSO-$d_6$) δ: 1.26(3H, t), 2.84(2H, q), 5.51(2H, s), 7.04(2H, d), 7.12(2H, d), 7.26 (1H, dd), 7.48-7.71(4H, m), 8.01(1H, dd), 8.31(1H, dd).

IR (KBr) cm$^{-1}$: 1600, 1500, 1460, 1420, 1400, 1280, 1250, 1040, 800, 775, 755.

Working Example 10

2-Ethyl-1-[[2'-(1H-tetrazol-5-yl)biphenyl-4-yl]methyl]imidazo[4,5-b]pyridine

The compound was prepared according to the procedure for Working Example 7.
Colorless crystals (13 %).
M.p. 141-143°C (dec.).

Elemental Analysis for $C_{22}H_{19}N_7 \cdot 0.1\ C_6H_{12}O$

| | C (%) | H (%) | N (%) |
|---|---|---|---|
| Calcd: | C, 67.15; | H, 5.44; | N, 24.25 |
| Found: | C, 67.28; | H, 5.36; | N, 23.99. |

$^1$H-NMR (200MHz, DMSO-$d_6$) δ: 1.29(3H, t), 2.88(2H, q), 5.52(2H, s), 7.06(4H, s), 7.19(1H, dd), 7.49-7.70(4H, m), 7.93(1H, dd), 8.35(1H, dd).

IR (KBr) cm$^{-1}$: 1605, 1510, 1480, 1455, 1410, 1280, 780, 755.

Working Example 11

2-Propyl-3-[[2'-(1H-tetrazol-5-yl)biphenyl-4-yl]methyl]imidazo[4,5-b]pyridine

The compound was prepared according to the procedure for Working Example 7.
Colorless plates (59 %).
M.p. 207-208°C (dec.).

Elemental Analysis for $C_{23}H_{21}N_7 \cdot 0.2\ H_2O$

| | C (%) | H (%) | N (%) |
|---|---|---|---|
| Calcd: | C, 69.22; | H, 5.40; | N, 24.57 |
| Found: | C, 69.59; | H, 5.35; | N, 24.38. |

$^1$H-NMR (200MHz, DMSO-$d_6$) δ: 0.93(3H, t), 1.72(2H, m), 2.80(2H, t), 5.52(2H, s), 7.05(2H, d), 7.12 (2H, d), 7.26(1H, dd), 7.50-7.72(4H, m), 8.01 (1H, dd), 8.28(1H, dd).

IR (KBr) cm$^{-1}$: 1600, 1505, 1465, 1410, 1280, 1225, 775, 760.

Working Example 12

2-Propyl-1-[[2'-(1H-tetrazol-5-yl)biphenyl-4-yl]methyl]imidazo[4,5-b]pyridine 4N-oxide

The compound was prepared according to the procedure for Working Example 7.
Colorless needles (28 %).
M.p. 192-195° C (dec.).

Elemental Analysis for $C_{23}H_{21}N_7O \cdot 0.7\ H_2O$

| | C (%) | H (%) | N (%) |
|---|---|---|---|
| Calcd: | C, 65.14; | H, 5.32; | N, 23.12 |
| Found: | C, 65.19; | H, 5.12; | N, 23.13. |

$^1$H-NMR (200MHz, DMSO-$d_6$) δ : 0.93(3H, t), 1.73(2H, m), 2.83(2H, t), 5.55(2H, s), 7.08(4H, s), 7.17-(1H, dd), 7.48-7.71(5H, m), 8.14(1H, d).
IR (KBr) $cm^{-1}$: 1450, 1410, 1245, 1220, 780, 735.

Working Example 13

2-Butyl-1-[[2'-(1H-tetrazol-5-yl)biphenyl]-methyl]imidazo[4,5-c]pyridine

The compound was prepared according to the procedure for Working Example 7.
Colorless leaflets (47 %).
M.p. 146-147° C.

Elemental Analysis for $C_{24}H_{23}N_7 \cdot 0.2\ H_2O$

| | C (%) | H (%) | N (%) |
|---|---|---|---|
| Calcd: | C, 69.78; | H, 5.71; | N, 23.73 |
| Found: | C, 69.88; | H, 5.69; | N, 23.49. |

$^1$H-NMR (200MHz, DMSO-$d_6$) δ : 0.87(3H, t), 1.27-1.45(2H, m), 1.63-1.78(2H, m), 2.84(2H, t), 5.53(2H, s), 7.06(4H, s), 7.46-7.69(5H, m), 8.29(1H, d), 8.89(1H, s).
IR (KBr) $cm^{-1}$: 1615, 1515, 1475, 1450, 1400, 825, 755.

Working Example 14

2-Butyl-3-[[2'-(1H-tetrazol-5-yl)biphenyl]-methyl]imidazo[4,5-c]pyridine

The compound was prepared according to the procedure for Working Example 7.
Colorless crystals (30 %).
M.p. 116-117° C.

Elemental Analysis for $C_{24}H_{23}N_7 \cdot 0.2\ H_2O$

| | C (%) | H (%) | N (%) |
|---|---|---|---|
| Calcd: | C, 69.78; | H, 5.71; | N, 23.73 |
| Found: | C, 69.49; | H, 5.83; | N, 23.91. |

$^1$H-NMR (200MHz, DMSO-$d_6$) δ : 0.92(3H, t), 1.34-1.52(2H, m), 1.75-1.90(2H, m), 2.93(2H, t), 5.38(2H, s), 6.59(2H, d), 6.75(2H, d), 7.17-7.42(3H, m), 7.71(1H, d), 7.77-7.82(1H, m), 8.24(1H, d), 9.00(1H, brs), 9.95(1H, brs).

| | |
|---|---|
| IR (KBr) $cm^{-1}$: | 1500, 1470, 1415, 825, 765. |

Working Example 15

2-Butyl-6-methyl-3-[[2'-(1H-tetrazol-5-yl)-biphenyl-4-yl]methyl]imidazo[5,4-b]pyridine

The compound was prepared according to the procedure for Working Example 7.
Colorless needles (77 %).
M.p. 136-140 °C.

Elemental Analysis for $C_{25}H_{25}N_7 \cdot 0.3\ H_2O$

| | C (%) | H (%) | N (%) |
|---|---|---|---|
| Calcd: | C, 70.01; | H, 6.02; | N, 22.86 |
| Found: | C, 70.01; | H, 5.96; | N, 22.61. |

| | |
|---|---|
| $^1$H-NMR (200MHz, $CDCl_3$) δ : | 0.86(3H, t), 1.22-1.40(2H, m), 1.55-1.70(2H, m), 2.30(2H, s), 2.47(2H, t), 5.35(2H, s), 6.78(2H, d), 6.91(2H, d), 6.99 (1H, d), 7.31-7.36(1H, m), 7.57-7.65(2H, m), 7.94-8.01(1H, m), 8.05(1H, d). |
| IR (KBr) $cm^{-1}$: | 1600, 1500, 1450, 1395, 880, 750. |

Working Example 16

2-Butyl-6-methyl-1-[[2'-(1H-tetrazol-5-yl)-biphenyl-4-yl]methyl]imidazo[5,4-b]pyridine

The compound was prepared according to the procedure for Working Example 7.
Colorless crystals (75 %).
M.p. 152-153 °C.

Elemental Analysis for $C_{25}H_{25}N_7 \cdot 0.4\ H_2O$

| | C (%) | H (%) | N (%) |
|---|---|---|---|
| Calcd: | C, 69.71; | H, 6.04; | N, 22.76 |
| Found: | C, 69.77; | H, 6.00; | N, 22.53. |

| | |
|---|---|
| $^1$H-NMR (200MHz, $CDCl_3$) δ : | 0.82(3H, t), 1.20-1.39(2H, m), 1.57-1.72(2H, m), 2.36(2H, s), 2.61(2H, t), 5.24(2H, s), 6.73(2H, d), 6.98(2H, d), 7.27-7.35(2H, m), 7.44-7.58(2H, m), 7.89(1H, dd), 8.05(1H, d). |
| IR (KBr) $cm^{-1}$: | 1615, 1560, 1515, 1485, 1450, 1410, 1280, 1000, 870, 785, 760. |

Working Example 17

5-Chloro-2-propyl-1-[[2'-(1H-tetrazol-5-yl)-biphenyl-4-yl]methyl]imidazo[4,5-b]pyridine

To a solution of 5-chloro-2-propyl-1-[[2'-(N-trityltetrazol-5-yl)biphenyl-4-yl]methyl]imidazo[4,5-b]pyridine (0.26 g) in methanol (8 ml) was added 1 N-HCl (0.6 ml) and the solution was allowed to stir at room temperature for 1 hour. The solution was concentrated to dryness and the resulting product was purified by column chromatography on silica gel to give pale yellow powders (0.12 g, 68 %).

Elemental Analysis for $C_{23}H_{20}ClN_7 \cdot 1.5\ H_2O$

| | C (%) | H (%) | N (%) |
|---|---|---|---|
| Calcd: | C, 60.46; | H, 5.07; | N, 21.46 |
| Found: | C, 60.12; | H, 4.59; | N, 21.18. |

Working Example 18

5-Chloro-2-propyl-3-[[2'-(1H-tetrazol-5-yl)-biphenyl-4-yl]-methyl]imidazo[4,5-b]pyridine

The compound was prepared according to the procedure for Working Example 17.
White powder (71 %).
M.p. 232-234° C.

Elemental Analysis for $C_{23}H_{20}ClN_7 \cdot 1/2\ H_2O$

| | C (%) | H (%) | N (%) |
|---|---|---|---|
| Calcd: | C, 62.94; | H, 4.82; | N, 22.34 |
| Found: | C, 63.24; | H, 4.62; | N, 22.17. |

$^1$H-NMR (200MHz, $CDCl_3$) δ : 0.87(3H, t), 1.59-1.78(2H, m), 2.57(2H, t), 5.32(2H, s), 6.85-7.00(4H, m), 7.14(1H, d), 7.30-7.38(1H, m), 7.43-7.61(3H, m), 7.83-7.91(1H, m).

Working Example 19

5-Dimethylamino-2-propyl-3-[[2'-(1H-tetrazol-5-yl)biphenyl-4-yl]methyl]imidazo[4,5-b]pyridine

The compound was prepared according to the procedure for Working Example 17.
Pale brown powder (57 %).
M.p. 264-266° C.

Elemental Analysis for $C_{25}H_{26}N_8 \cdot 1.1\ H_2O$

| | C (%) | H (%) | N (%) |
|---|---|---|---|
| Calcd: | C, 65.51; | H, 6.20; | N, 24.45 |
| Found: | C, 65.63; | H, 5.83; | N, 24.05. |

$^1$H-NMR (200MHz, $CDCl_3$) δ : 0.75(3H, t), 1.39-1.57(2H, m), 2.23(2H, t), 3.02(6H, s), 5.20(2H, s), 6.26-(1H, d), 6.87(1H, d), 6.82(2H, d), 6.95 (2H, d), 7.33-7.37(1H, m), 7.49-7.60(2H, m), 7.84-7.89(1H, m).

Working Example 20

2-Propyl-5-propylthio-3-[[2'-(1H-tetrazol-5-yl)biphenyl-4-yl]methyl]imidazo[4,5-b]pyridine

The compound was prepared according to the procedure for Working Example 17.

A pale yellow crystal (70 %).
M.p. 219-222° C.

Elemental Analysis for $C_{28}H_{27}N_7S \cdot 1/2\ H_2O$

| | C (%) | H (%) | N (%) |
|---|---|---|---|
| Calcd: | C, 65.25; | H, 5.90; | N, 20.49 |
| Found: | C, 64.95; | H, 5.71; | N, 20.32. |

$^1$H-NMR (200MHz, $CDCl_3$) δ : 0.83(3H, t), 0.91(3H, t), 1.50-1.70(4H,m), 2.39(2H, t), 3.04(2H, t), 5.32-(2H, s), 6.81-7.04(6H, m), 7.33-7.39(1H, m), 7.54-7.64(2H, m), 7.90-7.97(1H, m).

Working Example 21

2-Propyl-5-propylthio-1-[[2'-(1H-tetrazol-5-yl)biphenyl-4-yl]-methyl]imidazo[4,5-b]pyridine

The compound was prepared according to the procedure for Working Example 17.
A pale yellow powder (66 %).

Elemental Analysis for $C_{26}H_{27}N_7S \cdot 3/5\ H_2O$

| | C (%) | H (%) | N (%) |
|---|---|---|---|
| Calcd: | C, 65.00; | H, 5.92; | N, 20.41 |
| Found: | C, 65.00; | H, 5.62; | N, 20.18. |

$^1$H-NMR (200MHz, $CDCl_3$) δ : 0.83(3H, t), 0.95(3H, t), 1.54-1.73(4H,m), 2.52(2H, t), 3.04(2H, t), 5.15-(2H, s), 6.69(2H, d), 6.89(2H, d), 6.93 (1H, d), 7.22-7.53(4H, m), 7.74-7.81(1H, m).

Working Example 22

5-Methoxy-2-propyl-1-[[2'-(1H-tetrazol-5-yl)-biphenyl-4-yl]methyl]imidazo[4,5-b]pyridine

The compound was prepared according to the procedure for Working Example 7.
Colorless prisms (66 %).
M.p. 191-192° C (dec.).

Elemental Analysis for $C_{24}H_{23}N_7O \cdot 1/2\ H_2O$

| | C (%) | H (%) | N (%) |
|---|---|---|---|
| Calcd: | C, 66.34; | H, 5.57; | N, 22.57 |
| Found: | C, 66.44; | H, 5.42; | N, 22.62. |

$^1$H-NMR (200MHz, $CDCl_3$) δ : 0.94(3H, t), 1.71(2H, m), 2.53(2H,t), 3.61(3H, s), 5.25(2H, s), 6.54(1H, d), 6.71(2H, d), 6.97(2H, d), 7.25-7.34(2H, m), 7.46-7.59(2H, m), 7.91-7.95(1H, m).

IR (KBr) $cm^{-1}$: 1600, 1590, 1500, 1480, 1450, 1435, 1405, 1360, 1340, 1295, 1225,

1200, 1035, 805, 755.

Working Example 23

5-Methoxy-2-propyl-3-[[2'-(1H-tetrazol-5-yl)-biphenyl-4-yl]methyl]imidazo[4,5-b]pyridine

The compound was prepared according to the procedure for Working Example 7.
Colorless prisms (65 %).
M.p. 258-260 °C (dec.).

Elemental Analysis for $C_{24}H_{23}N_7O \cdot 0.3\ H_2O$

| | C (%) | H (%) | N (%) |
|---|---|---|---|
| Calcd: | C, 66.90; | H, 5.50; | N, 22.75 |
| Found: | C, 67.06; | H, 5.40; | N, 22.90. |

$^1$H-NMR (200MHz, $CDCl_3$) δ : 0.92(3H, t), 1.69(2H, m), 2.74(2H,t), 3.89(3H, s), 5.42(2H, s), 6.67(1H, d), 7.06(2H, d), 7.19(2H, d), 7.49-7.72(4H, m), 7.91(1H, d).

IR (KBr) $cm^{-1}$ : 1600, 1590, 1455, 1435, 1405, 1355, 1345, 1260, 1200, 1100, 1020, 1005, 875, 850, 815, 775, 745, 725.

Working Example 24

6-Bromo-2-butyl-5-methyl-3-[[2'-(1H-tetrazol-5-yl)biphenyl-4-yl]methyl]imidazo[5,4-b]pyridine

The compound was prepared according to the procedure for Working Example 17.
Colorless prisms (76 %).
M.p. 230-231 °C (dec.).

Elemental Analysis for $C_{25}H_{24}BrN_7$

| | C (%) | H (%) | N (%) |
|---|---|---|---|
| Calcd: | C, 59.77; | H, 4.81; | N, 19.52 |
| Found: | C, 59.70; | H, 4.71; | N, 19.62. |

$^1$H-NMR (200MHz, $CDCl_3$) δ : 0.84(3H, t), 1.19-1.38(2H, m), 1.53-1.68(2H, m), 2.49(2H,t), 2.66(3H, s), 5.38(2H, s), 6.86(2H, d), 6.97(2H, d), 7.33-7.39(2H, m), 7.57-7.68(2H, m), 7.96-8.00(1H, m).

IR (KBr) $cm^{-1}$: 1595, 1575, 1500, 1460, 1400, 1360, 1270, 990, 945, 895, 770, 745, 725.

Working Example 25

6-Bromo-2-butyl-5-methyl-1-[[2'-(1H-tetrazol-5-yl)biphenyl-4-yl]methyl]imidazo[5,4-b]pyridine

The compound was prepared according to the procedure for Working Example 17.
Colorless prisms (54 %).
M.p. 141-143 °C (dec.).

Elemental Analysis for $C_{25}H_{24}N_7Br \cdot 0.1\ C_4H_8O_2$

| | C (%) | H (%) | N (%) |
|---|---|---|---|
| Calcd: | C, 59.68; | H, 4.89; | N, 19.18 |
| Found: | C, 59.64; | H, 5.01; | N, 18.87. |

$^1$H-NMR (200MHz, $CDCl_3$) δ : 0.88(3H, t), 1.24-1.45(2H, m), 1.62-1.77(2H, m), 2.51(3H, s), 2.64(2H, t), 5.25(2H, s), 6.73(2H, d), 6.95(2H, d), 7.27-7.33(1H, m), 7.51-7.61(3H, m), 7.94-7.99(1H, m).

IR (KBr) $cm^{-1}$: 1600, 1500, 1480, 1450, 1400, 1370, 1275, 970, 775, 755.

Working Example 26

5-Cyano-2-propyl-1-[[2'-(1H-tetrazol-5-yl)-biphenyl-4-yl]methyl]imidazo[4,5-b]pyridine

The compound was prepared according to the procedure for Working Example 7.
Colorless crystals (91 %).
M.p. 233-234 °C (dec.).

Elemental Analysis for $C_{24}H_{20}N_8 \cdot 0.4\ H_2O$

| | C (%) | H (%) | N (%) |
|---|---|---|---|
| Calcd: | C, 67.40; | H, 4.90; | N, 26.20 |
| Found: | C, 67.44; | H, 4.76; | N, 25.96. |

$^1$H-NMR (200MHz, DMSO-$d_6$) δ : 0.95(3H, t), 1.76(2H, m), 2.88(2H, t), 5.61(2H, s), 7.08(4H, s), 7.47-7.71(4H, m), 7.84(1H, d), 8.18(1H, d).

IR (KBr) $cm^{-1}$: 2200, 1600, 1500, 1480, 1440, 1410, 1300, 990, 820, 750.

Working Example 27

5-Cyano-2-propyl-3-[[2'-(1H-tetrazol-5-yl)-biphenyl-4-yl]methyl]imidazo[4,5-b]pyridine

The compound was prepared according to the procedure for Working Example 7.
Colorless crystals (71 %).
M.p. 233-234 °C (dec.).

Elemental Analysis for $C_{24}H_{20}N_8$

| | C (%) | H (%) | N (%) |
|---|---|---|---|
| Calcd: | C, 68.56; | H, 4.79; | N, 26.65 |
| Found: | C, 68.64; | H, 5.03; | N, 26.56. |

$^1$H-NMR (200MHz, DMSO-$d_6$) δ : 0.93(3H, t), 1.73(2H, m), 2.85(2H, t), 5.56(2H, s), 7.06(2H, d), 7.13-(2H, d), 7.49-7.71(4H, m), 7.89(1H, d), 8.23(1H, d).

IR (KBr) $cm^{-1}$: 2250, 1600, 1570, 1500, 1475, 1460, 1405, 1370, 1270, 1240, 1220, 1090, 1060, 995, 930, 830, 770, 760.

Working Example 28

Ethyl 2-propyl-1-[[2'-(1H-tetrazol-5-yl)-biphenyl-4-yl]methyl]imidazo[4,5-b-]pyridine-5-carboxylate

A solution of 5-cyano-2-propyl-1-[[2'-(1H-tetrazol-5-yl)biphenyl-4-yl]methyl]imidazo[4,5-b]-pyridine (1.0 g) in 15% ethanolic hydrogen chloride (10 ml) was heated at reflux for 2.5 hours, and then it was evaporated to dryness to give a residue. The resulting product was purified by column chromatography on silica gel to give a crystalline product. Recrystallization from ethyl acetate-methanol gave colorless needles (0.77 g, 64 %).
M.p. 164-166 °C (dec.).

Elemental Analysis for $C_{26}H_{25}N_7O_2 \cdot 1/4\ C_4H_8O_2$

| | C (%) | H (%) | N (%) |
|---|---|---|---|
| Calcd: | C, 65.05; | H, 5.66; | N, 19.67 |
| Found: | C, 65.28; | H, 5.36; | N, 19.70. |

$^1$H-NMR (200MHz, DMSO-$d_6$) δ : 0.96(3H, t), 1.36(3H, t), 1.78(2H, m), 2.88(2H, t), 4.36(2H, q), 5.59-(2H, s), 7.07(4H, s), 7.48-7.71(4H, m), 7.97(1H, d), 8.07(1H, d).

IR (KBr) cm$^{-1}$: 1725, 1445, 1420, 1400, 1300, 1255, 1120, 760.

Working Example 29

Ethyl 2-propyl-3-[[2'-(1H-tetrazol-5-yl)-biphenyl-4-yl]methyl]imidazo[4,5-b]pyridine-5-carboxylate

The compound was prepared according to the procedure for Working Example 28.
Colorless prisms (92 %).
M.p. 212-213 °C (dec.).

Elemental Analysis for $C_{28}H_{25}N_7O_2$

| | C (%) | H (%) | N (%) |
|---|---|---|---|
| Calcd: | C, 66.79; | H, 5.39; | N, 20.97 |
| Found: | C, 66.89; | H, 5.37; | N, 20.86. |

$^1$H-NMR (200MHz, DMSO-$d_6$) δ : 0.92(3H, t), 1.35(3H, t), 1.73(2H, m), 2.81(2H, t), 4.38(2H, q), 5.57-(2H, s), 7.06(2H, d), 7.14(2H, d), 7.50-7.71(4H, m), 8.03(1H, d), 8.16(1H, d).

IR (KBr) cm$^{-1}$: 1710, 1600, 1500, 1460, 1410, 1370, 1280, 1260, 1235, 1120, 860, 760.

Working Example 30

2-Propyl-1-[[2'-(1H-tetrazol-5-yl)biphenyl-4-yl]methyl]imidazo[4,5-b]pyridine-5-carboxylic acid

A solution of ethyl 2-propyl-1-[[2'-(1H-tetrazol-5-yl)biphenyl-4-yl]methyl]imidazo[4,5-b]-pyridine-5-carboxylate (0.4 g) in methanol (5 ml) containing 1 N aqueous sodium hydroxide (2.6 ml) was heated at reflux for 2 hours. The reaction solution was evaporated to dryness to give a residue, which was dissolved in water. The solution was acidified by addition of 1 N HCl (2.6 ml) to give a crystalline precipitate. Recrystallization from methanol gave colorless needles (0.2 g, 53 %).
M.p. 211-213 °C.

Elemental Analysis for $C_{24}H_{21}N_7O_2 \cdot 1.7\ H_2O$

| | C (%) | H (%) | N (%) |
|---|---|---|---|
| Calcd: | C, 61.32; | H, 5.23; | N, 20.86 |
| Found: | C, 61.52; | H, 5.05; | N, 20.62. |

$^1$H-NMR (200MHz, DMSO-$d_6$) δ : 0.96(3H, t), 1.78(2H, m), 2.88(2H, t), 5.59(2H, s), 7.08(4H, s), 7.48-7.71(4H, m), 7.97(1H, d), 8.07(1H, d).

IR (KBr) $cm^{-1}$: 1720, 1600, 1460, 1410, 1380, 1320, 1300, 1235, 760, 740.

Working Example 31

2-Propyl-3-[[2'-(1H-tetrazol-5-yl)biphenyl-4-yl]methyl]imidazo[4,5-b]pyridine-5-carboxylic acid

The compound was prepared according to the procedure for Working Example 30.
Colorless needles (46 %).
M.p. 151-152 °C.

Elemental Analysis for $C_{24}H_{21}N_7O_2 \cdot 0.5\ H_2O$

| | C (%) | H (%) | N (%) |
|---|---|---|---|
| Calcd: | C, 64.28; | H, 4.94; | N, 21.86 |
| Found: | C, 64.61; | H, 5.25; | N, 21.51. |

$^1$H-NMR (200MHz, DMSO-$d_6$) δ : 0.92(3H, t), 1.72(2H, m), 2.81(2H, t), 5.59(2H, s), 7.05(2H, d), 7.13-(2H, d), 7.49-7.71(4H, m), 8.01(1H, d), 8.14(1H, d).

IR (KBr) $cm^{-1}$: 1720, 1600, 1500, 1480, 1455, 1410, 1380, 1310, 1260, 755.

Working Example 32

4-Cyano-2-propyl-1-[[2'-(1H-tetrazol-5-yl)-biphenyl-4-yl]methyl]imidazo[4,5-c]pyridine

The compound was prepared according to the procedure for Working Example 17.
Colorless needles (87 %).
M.p. 225-230 °C (dec.).

Elemental Analysis for $C_{24}H_{20}N_8 \cdot 3.4\ H_2O$

| | C (%) | H (%) | N (%) |
|---|---|---|---|
| Calcd: | C, 59.84; | H, 5.61; | N, 23.26 |
| Found: | C, 59.97; | H, 5.34; | N, 23.17. |

$^1$H-NMR (200MHz, DHSO-$d_6$) δ : 0.98(3H, t), 1.81(2H, m), 2.94(2H, t), 5.59(2H, s), 6.98(2H, d), 7.10-(2H, d), 7.23-7.67(3H, m), 7.52-7.57(1H, m), 7.95 (1H, d), 8.46(1H, d).

IR (KBr) $cm^{-1}$: 2220, 1600, 1580, 1500, 1450, 1440, 1400, 1350, 1270, 1200, 830, 810, 780, 755.

Working Example 33

4-Cyano-2-propyl-3-[[2'-(1H-tetrazol-5-yl)-biphenyl-4-yl]methyl]imidazo[4,5-c]pyridine

The compound was prepared according to the procedure for Working Example 17.
Colorless prisms (62 %).
M.p. 242-243 ° C (dec.).

Elemental Analysis for $C_{24}H_{20}N_8$

| | C (%) | H (%) | N (%) |
|---|---|---|---|
| Calcd: | C, 68.56; | H, 4.79; | N, 26.65 |
| Found: | C, 68.17; | H, 4.50; | N, 26.39. |

$^1$H-NMR (200MHz, DMSO-$D_6$) δ : 0.93(3H, t), 1.75(2H, m), 2.86(2H, t), 5.78(2H, s), 7.01(2H, d), 7.09-(2H, d), 7.51-7.71(4H, m), 8.02(1H, d), 8.52(1H, d).
IR (KBr) cm$^{-1}$: 2220, 1600, 1565, 1500, 1460, 1425, 1405, 1270, 1190, 1135, 985, 840, 770, 750.

Working Example 34

Ethyl 2-propyl-1-[[2'-(1H-tetrazol-5-yl)-biphenyl-4-yl]methyl]imidazo[4,5-c]pyridine-5-carboxylate

The compound was prepared according to the procedure for Working Example 28.
Colorless leaflets (70 %).
M.p. 218-219 ° C (dec.).

Elemental Analysis for $C_{26}H_{25}N_7O_2 \cdot 0.2\ H_2O$

| | C (%) | H (%) | N (%) |
|---|---|---|---|
| Calcd: | C, 66.29; | H, 5.43; | N, 20.81 |
| Found: | C, 66.16; | H, 5.42; | N, 20.97. |

$^1$H-NMR (200MHz, DMSO-$d_6$) δ : 0.95(3H, t), 1.35(3H, t), 1.73(2H, m), 2.86(2H, t), 4.39(2H, q), 5.59-(2H, s), 7.06(4H, s), 7.48-7.71(4H, m), 7.80(1H, d), 8.35(1H, d).
IR (KBr) cm$^{-1}$: 1730, 1600, 1460, 1440, 1400, 1305, 1235, 1180, 1145, 835, 755.

Working Example 35

2-Propyl-1-[[2'-(1H-tetrazol-5-yl)biphenyl-4-yl]methyl]imidazo[4,5-c]pyridine-5-carboxylic acid

The compound was prepared according to the procedure for Working Example 30.
Colorless needles (78 %).
M.p. 179-181 ° C (dec.).

Elemental Analysis for $C_{24}H_{21}N_7O_2 \cdot 0.8\ C_3H_7NO \cdot 0.5\ H_2O$

| | C (%) | H (%) | N (%) |
|---|---|---|---|
| Calcd: | C, 62.54; | H, 5.49; | N, 21.55 |
| Found: | C, 62.25; | H, 5.22; | N, 21.42. |

$^1$H-NMR (200MHz, DMSO-$d_6$) δ : 0.95(3H, t), 1.73(2H, m), 2.87(2H, t), 5.60(2H, s), 7.07(4H, s), 7.48-7.71(4H, m), 7.81(1H, d), 8.37(1H, d).

IR (KBr) $cm^{-1}$: 1650, 1605, 1405, 1370, 1350.

Experimental Example 1

Inhibition of binding of angiotensin-II to angiotensin receptor

[Method]

An experiment of inhibition on the binding of angiotensin-II (A-II) to A-II-receptor was conducted by modifying the method of Douglas et al. [Endocrinology, 102, 685-696 (1978)]. An A-II-receptor was prepared from the membrane fraction of bovine adrenal cortex.

The compound of the present invention ($10^{-7}$M or $10^{-6}$M ) and $^{125}$I-A-II (1.85 kBq/50 μl) were added to the receptor membrane fraction, and the mixture was incubated at room temperature for one hour. The receptor-bound and free $^{125}$I-A-II were separated through a filter (Whatman GF/B filter), and the radioactivity of $^{125}$I-A-II bound to the receptor was measured.

[Results]

The results relating to the compounds of the present invention are shown in Table 1.

Experimental Example 2

Inhibitory effect of the compound of the present invention on pressor action of A-II

[Method]

Jcl : SD rats (9 week old, male) were used. On the day previous to the experiment, these animals were applied with cannulation into the femoral artery and vein under anesthesia with pentobarbital Na. The animals were fasted but allowed to access freely to drinking water until the experiment was started. Just on the day of conducting the experiment, the artery cannula was connected with a blood-pressure transducer, and the average blood pressure was recorded by means of polygraph. Before administration of the drug, the pressor action due to intravenous administration of A-II (100 ng/kg) as the control was measured. The drugs were orally administered, and then, at each point of the measurement, A-II was administered intravenously, and the pressor action was similarly measured. By comparing the pressor action before and after administration of the drug, the percent inhibition by the drug on A-II-induced pressor action was evaluated.

[Results]

The results relating to the compounds of the present invention are shown in Table 1.

TABLE 1

| Working Example No. | Radioreceptor Assay (%) | | Pressor Response to A II (p.o.) | |
|---|---|---|---|---|
| | $1X10^{-7}M$ | $1X10^{-6}M$ | 10 mg/kg | 30 mg/kg |
| 3 | 63 | 92 | ++ *1 | +++ |
| 4 | 5 | 48 | NT *2 | NT |
| 6 | 43 | 81 | NT | NT |
| 9 | 50 | 84 | NT | +++ |
| 13 | 1 | 44 | NT | NT |
| 14 | 9 | 56 | NT | NT |
| 15 | 32 | 75 | NT | NT |
| 16 | 0 | 32 | NT | NT |
| 23 | 32 | 75 | NT | NT |
| 24 | 0 | 9 | NT | NT |

*1 : +++ ≧ 70 % > ++ ≧ 50 %.

*2 : NT , not tested.

It is understood that the preceding representative examples may be varied within the scope of the present invention by one skilled in the art to achieve essentially the same results.

As many widely different embodiments of this invention may be made without departing from the spirit and scope thereof, it is to be understood that this invention is not limited to the specific embodiments thereof except as defined in the appended claims.

**Claims**

1. A compound of the formula:

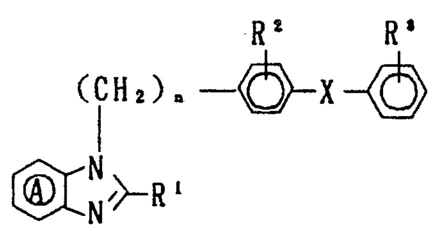

wherein the ring A, which may be optionally substituted, is a six-membered heteroaromatic group having one or two hetero nitrogen atoms; $R^1$ is hydrogen or an alkyl, aralkyl or aryl group which may be optionally substituted;

$R^2$ is hydrogen, halogen or nitro;
$R^3$ is a residue capable of forming an anion or a residue convertible into an anion;
X is a direct bond or a spacer having atomic length of two or less; and
n is an integer of 1 or 2;
and a pharmaceutically acceptable salt thereof.

2. A compound according to claim 1, wherein $R^1$ is lower alkyl of 1 to about 8 carbon atoms, which may be straight or branched, and may be optionally substituted with hydroxyl, optionally substituted amino, halogen, or lower ($C_{1-4}$) alkoxy.

3. A compound according to claim 1, wherein $R^1$ is phenyl-lower ($C_{1-4}$) alkyl optionally substituted with halogen, nitro, lower ($C_{1-4}$) alkoxy, or lower ($C_{1-4}$) alkyl.

4. A compound according to claim 1, wherein $R^1$ is phenyl or naphtyl optionally substituted with halogen, nitro, lower ($C_{1-4}$) alkoxy, or lower ($C_{1-4}$) alkyl.

5. A compound according to claim 1, wherein the ring A is a six-membered heteroaromatic group having one hetero nitrogen atom or a six-membered heteroaromatic group having two hetero nitrogen atoms.

6. A compound according to claim 1, wherein the ring A is pyridine, pyrazine, pyrimidine, or pyridazine.

7. A compound according to claim 1, wherein the ring A is pyridine or pyrazine.

8. A compound according to claim 1, wherein the ring A is optionally substituted with halogen, cyano, optionally substituted amino, -Y-R wherein Y is a bond, -O-, -S- or -(C=O)-, and R is lower alkyl which may be optionally substituted with hydroxyl, optionally substituted amino, halogen or lower ($C_{1-4}$) alkoxy, or -COD wherein D is lower ($C_{1-4}$) alkoxy which may be optionally substituted with hydroxyl, optionally substituted amino, halogen or lower ($C_{1-4}$) alkoxy on the alkyl portion, optionally substituted amino, halogen or OH.

9. A compound according to claim 8, wherein the optionally substituted amino is N-lower ($C_{1-4}$) alkyl amino, N,N-dilower ($C_{1-4}$) alkyl amino, N-arylamino, or alicyclic amino.

10. A compound according to claim 9, wherein the alicyclic amino is morpholino, piperidino, piperazino, or N-phenylpiperazino.

11. A compound according to claim 9, wherein the N-arylamino is phenylamino.

12. A compound according to claim 1, wherein $R^3$ is carboxyl, lower ($C_{1-4}$) alkoxycarbonyl, cyano, tetrazolyl, trifluoromethanesulfonic amide ($-NHSO_2CF_3$), phosphoric acid, or sulfonic acid.

13. A compound according to claim 1, wherein $R^3$ is in the ortho position.

14. A compound according to claim 1, wherein $R^3$ is, in the ortho position, carboxyl, lower ($C_{1-4}$) alkoxycarbonyl, cyano or tetrazolyl.

15. A compound according to claim 1, wherein the group X is a direct bond between a phenylene group and a phenyl group.

16. A compound according to claim 1, wherein the group X is a spacer having atomic length of two or less wherein the constituent number of atoms in a straight chain is 1 or 2 and which may be branched.

17. A compound according to claim 1, wherein the group X is a spacer selected from the class consisting of lower ($C_{1-4}$) alkylene, -C(=O)-, -O-, -S-, -NH-, -C(=O)-NH-, $-O-CH_2-$, $-S-CH_2-$, and -CH=CH-.

18. A compound according to claim 1, which is a compound of the formula (I')

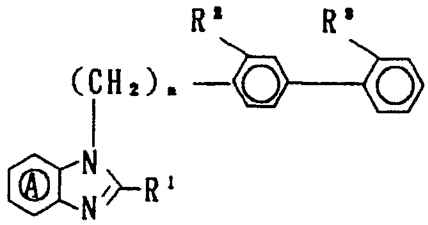

(I')

wherein the ring A is pyridine or pyrazine, which may be optionally substituted with one to three halogens, lower ($C_{1-4}$) alkoxy, nitro, amino, N-lower ($C_{1-4}$) alkyl amino, N,N-dilower ($C_{1-4}$) alkyl amino or -COD wherein D is hydroxyl or lower ($C_{1-4}$) alkoxy; $R^1$ is $C_{2-5}$ alkyl; $R^2$ is hydrogen; and $R^3$ is carboxyl or tetrazolyl; or a pharmaceutically acceptable salt thereof.

**19.** A compound according to claim 1 or a pharmaceutically acceptable salt thereof, which is 2-butyl-3-[[2'-(1H-tetrazole-5-yl)biphenyl-4-yl]-methyl]imidazo[4,5-b]pyridine.

**20.** A compound according to claim 1 or a pharmaceutically acceptable salt thereof, which is 2-ethyl-3-[[2'-(1H-tetrazole-5-yl)biphenyl-4-yl]-methyl]imidazo[4,5-b]pyridine.

**21.** A pharmaceutical composition for antagonizing angiotensin II which comprises a therapeutically effective amount of a compound according to claim 1 or a pharmaceutically acceptable salt thereof in admixture with a pharmaceutical acceptable carrier, excipient or diluent.

**22.** A use of a compound according to claim 1 or a pharmaceutically acceptable salt thereof for the manufacture of a medicament for antagonizing angiotensin II.

**23.** A method for producing a compound of the formula (I):

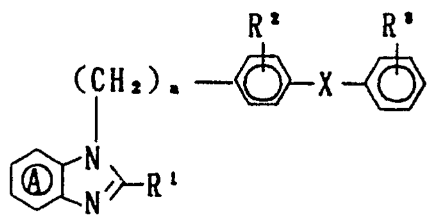

(I)

wherein the ring A, which may be optionally substituted, is a six-membered heteroaromatic group having one or two hetero nitrogen atoms; $R^1$ is hydrogen or an alkyl, aralkyl or aryl group which may be optionally substituted;
$R^2$ is hydrogen, halogen or nitro;
$R^3$ is a residue capable of forming an anion or a residue convertible into an anion;
X is a direct bond or a spacer having atomic length of two or less; and
n is an integer of 1 or 2;
and a pharmaceutically acceptable salt thereof,
which comprises
reacting a compound of the formula (II):

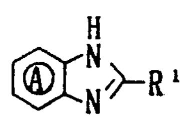

II

wherein A, $R^1$, $R^2$, $R^3$, X and n have the above-defined meanings, with a compound of the formula (III):

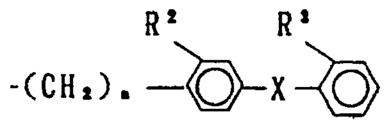

III

wherein A, $R^1$, $R^2$, $R^3$, X and n have the above-defined meanings and W is halogen,
and, (i) if desired, converting a compound of the formula (I) wherein $R^3$ is cyano or protected tetrazolyl, and A, $R^1$, $R^2$, X and n have the above-defined meanings, into a compound of the formula (I) wherein $R^3$ is tetrazolyl and A, $R^1$, $R^2$, X and n have the above-defined meanings, or (ii) if desired, converting a compound of the formula (I) wherein $R^3$ is an ester, and A, $R^1$, $R^2$, X and n have the above-defined meanings, into a compound of the formula (I) wherein $R^3$ is carboxyl and A, $R^1$, $R^2$, X and n have the above-defined meanings, and, if desired, converting a compound of the formula (I) into a pharmaceutically acceptable salt thereof.

European Patent Office

# EUROPEAN SEARCH REPORT

Application Number

EP 90124780.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US - A - 4 766 130 (AUSTEL) * Abstract; column 12, lines 1-21 * | 1,21, 22 | C 07 D 471/04<br>C 07 D 487/04<br>C 07 D 473/00<br>A 61 K 31/435<br>A 61 K 31/495 |
| A | US - A - 4 816 463 (BLANKLEY) * Column 2, formula I; column 17, lines 14-28 * | 1,21, 22 | |
| A | EP - A2/A3 - 0 260 613 (G.D. SEARLE) * Abstract; page 22; claim 1 * | 1,21, 22 | |
| P,A | CHEMICAL ABSTRACTS, vol. 112, no. 21, May 21, 1990, Columbus, Ohio, USA CARINI et al. "Part VI. Nonpeptide angiotensin II receptor antagonists: N-((benzyloxy)benzyl) imidazoles and related compounds as potent antihypertensives" page 16, column 2, abstract-No. 191 374p & J.Med.Chem. 1990, 33(5), 1330-6 | 1,21, 22 | TECHNICAL FIELDS SEARCHED (Int. Cl.5)<br>C 07 D 471/00<br>C 07 D 487/00<br>C 07 D 473/00<br>A 61 K 31/00<br>C 07 D 233/00<br>C 07 D 235/00 |
| A | EP - A2 - 0 253 310 (E.I.DU PONT DE NEMOURS) * Page 5, lines 13-30; claims 5,6 * | 1, 21-23 | |
| A | EP - A2 - 0 186 190 (SUMITOMO) * Abstract * | 1 | |

The present search report has been drawn up for all claims

| Place of search | Date of completion of the search | Examiner |
|---|---|---|
| VIENNA | 12-02-1991 | ONDER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)